(19) Europäisches Patentamt
European Patent Office
Office européen des brevets

(11) Veröffentlichungsnummer : **0 361 067 B1**

(12) # EUROPÄISCHE PATENTSCHRIFT

(45) Veröffentlichungstag der Patentschrift : **11.11.92 Patentblatt 92/46**

(51) Int. Cl.$^5$ : **C07C 251/32,** C07C 49/175, C07C 323/47, C07C 323/22, C07C 251/76, C07D 213/64, A01N 35/00

(21) Anmeldenummer : **89115319.9**

(22) Anmeldetag : **19.08.89**

(54) **Disubstituierte Naphthaline, Verfahren zur Herstellung sowie deren herbizide Verwendung.**

(30) Priorität : **01.09.88 DE 3829586**

(43) Veröffentlichungstag der Anmeldung : **04.04.90 Patentblatt 90/14**

(45) Bekanntmachung des Hinweises auf die Patenterteilung : **11.11.92 Patentblatt 92/46**

(84) Benannte Vertragsstaaten : **BE CH DE ES FR GB IT LI NL**

(56) Entgegenhaltungen :
**EP-A- 0 023 891**
**EP-A- 0 200 686**
**CH-A- 670 084**
**DE-A- 3 220 524**

(73) Patentinhaber : **BAYER AG**
**W-5090 Leverkusen 1 Bayerwerk (DE)**

(72) Erfinder : **Andree, Roland, Dr.**
**Schillerstrasse 17**
**W-4018 Langenfeld (DE)**
Erfinder : **Haug, Michael, Dr.**
**Fahner Weg 5**
**W-5060 Bergisch-Gladbach 2 (DE)**
Erfinder : **Santel, Hans-Joachim, Dr.**
**Grünstrasse 9a**
**W-5090 Leverkusen 1 (DE)**
Erfinder : **Schmidt, Robert R., Dr.**
**Im Waldwinkel 110**
**W-5060 Bergisch-Gladbach 2 (DE)**
Erfinder : **Strang, Harry, Dr.**
**Unterdorfstrasse 6A**
**W-4000 Düsseldorf 31 (DE)**

EP 0 361 067 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue disubstituierte Naphthaline, Verfahren zu ihrer Herstellung und ihre Verwendung als Herbizide.

Es ist bereits bekannt, daß bestimmte Dioxy-benzol-Derivate, wie z. B. α-[4-(2,4-Dichlor-phenoxy)-phenoxy]-propionsäure-methylester (Diclofop-methyl), herbizid wirksam sind (vgl. DE-OS 22 23 894). Die Wirkung dieser bekannten Verbindungen gegen Unkräuter sowie ihre Kulturpflanzen-Verträglichkeit sind jedoch nicht immer zufriedenstellend.

Es wurden nun neue disubstituierte Naphthaline der allgemeinen Formel (I)

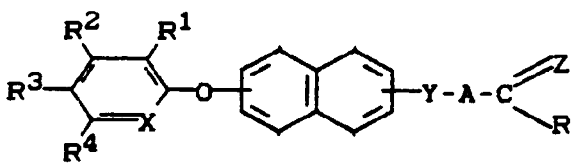

(I)

in welcher

A für gegebenenfalls verzweigtes Alkandiyl steht,

R für Wasserstoff oder für jeweils gegebenenfalls substituiertes Alkyl oder Phenyl steht,

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht,

X für Stickstoff oder die Gruppierung C-$R^5$ steht, worin

$R^5$ für Wasserstoff oder Halogen steht,

Y für Sauerstoff oder Schwefel steht, und

Z für Sauerstoff, die Gruppierung N-$(O)_n$-$R^6$ oder

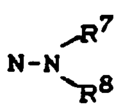

steht, worin

n für die Zahlen 0 oder 1 steht und

$R^6$ für Wasserstoff oder für einen gegebenenfalls substituierten Rest aus der Reihe Alkyl, Alkenyl, Alkinyl, Cycloalkyl, Cycloalkylalkyl, Aralkyl und Aryl steht,

$R^7$ für Wasserstoff oder Alkyl steht und

$R^8$ für Wasserstoff oder einen gegebenenfalls substituierten Rest aus der Reihe Alkyl, Alkenyl, Alkinyl, Cycloalkyl, Aralkyl, Aryl, Alkylcarbonyl, Alkoxycarbonyl, Alkylsufonyl, Arylsulfonyl und Heteroaryl steht,

gefunden.

Weiter wurde gefunden, daß man die neuen disubstituierten Naphthaline der allgemeinen Formel (I) erhält, wenn man

(a) substituierte Hydroxy- bzw. Mercaptonaphthaline der allgemeinen Formel (II)

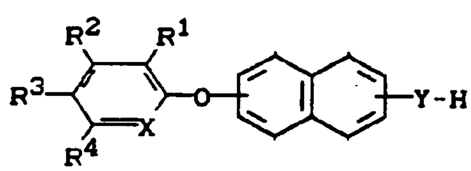

(II)

in welcher

$R^1$, $R^2$, $R^3$, $R^4$, X und Y die oben angegebenen Bedeutungen haben,

mit Alkylierungsmitteln der allgemeinen Formel (III)

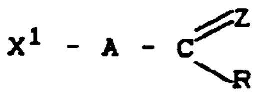

(III)

in welcher
A, R und Z die oben angegebenen Bedeutungen haben und
$X^1$ für Halogen steht,
- im Fall, daß Z für Sauerstoff steht, gegebenenfalls mit entsprechenden Dimethyl- oder Diethylacetalen bzw. -ketalen -
gegebenenfalls in Gegenwart eines Säureakzeptors und gegebenenfalls in Gegenwart eines Verdünnungsmittels umsetzt, oder wenn man
(b) für den Fall, daß Z für die Gruppierung N-$(O)_n$-$R^6$ oder für die Gruppierung

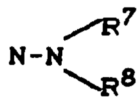

steht,
entsprechende Carbonylverbindungen der Formel (Ia)

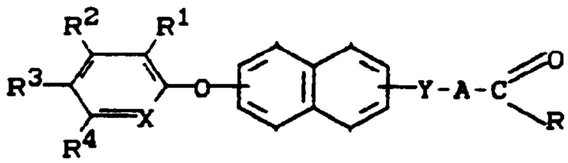

(Ia)

in welcher
A, R, $R^1$, $R^2$, $R^3$, $R^4$, X und Y die oben angegebenen Bedeutungen haben,
mit Aminoverbindungen der Formeln (IV) oder (V)

$H_2N-(O)_n-R^6$

(IV)

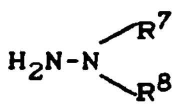

(V)

worin
n, $R^6$, $R^7$ und $R^8$ die oben angegebenen Bedeutungen haben,
oder mit deren Hydrochloriden
gegebenenfalls in Gegenwart eines Katalysators und gegebenenfalls in Gegenwart eines Verdünnungsmittels umsetzt, oder wenn man
(c) für den Fall, daß Z für die Gruppierung N-O-$R^6$ steht,
entsprechende Hydroximinoverbindungen der Formel (Ib)

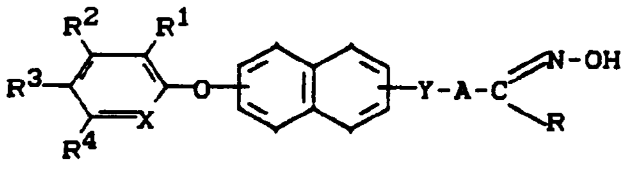

(Ib)

in welcher
A, R, $R^1$, $R^2$, $R^3$, $R^4$, X und Y die oben angegebenen Bedeutungen haben,
mit Alkylierungsmitteln der Formel (VI)

$X^2$ - $R^6$ (VI)

in welcher

$R^6$ die oben angegebene Bedeutung hat und

$X^2$ für Halogen, Alkylsulfonyloxy oder Arylsulfonyloxy steht,

gegebenenfalls in Gegenwart eines Verdünnungsmittels umsetzt.

Schließlich wurde gefunden, daß die neuen disubstituierten Naphthaline der allgemeinen Formel (I) herbizide Eigenschaften aufweisen.

Überraschenderweise zeigen die erfindungsgemäßen disubstituierten Naphthaline der Formel (I) bei guter Kulturpflanzen-Verträglichkeit gegen Problemunkräuter wesentlich stärkere Wirkung als α-(4-(2,4-Dichlorphenoxy)-phenoxy)-propionsäure-methylester, welches ein strukturell ähnlicher vorbekannter Wirkstoff gleicher Wirkungsrichtung ist.

Die Kohlenstoffketten in den einzelnen Resten, wie beispielsweise Alkyl, Alkenyl, Alkinyl, Cycloalkyl, Cycloalkylalkyl, Alkylcarbonyl, Alkoxycarbonyl oder Alkylsulfonyl, sind jeweils geradkettig oder verzweigt.

Die Erfindung betrifft vorzugsweise Verbindungen der Formel (I), in welcher

A für gegebenenfalls verzweigtes $C_1$-$C_4$-Alkandiyl steht,

R für Wasserstoff, $C_1$-$C_6$-Alkyl oder Phenyl steht,

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff, Fluor oder Chlor steht,

X für Stickstoff oder die Gruppierung C-$R^5$ steht, worin

$R^5$ für Wasserstoff, Fluor, Chlor oder Brom steht,

Y für Sauerstoff oder Schwefel steht, und

Z für Sauerstoff, die Gruppierung N-$(O)_n$-$R^6$ oder

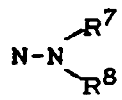

steht, worin

n für die Zahlen 0 oder 1 steht und

$R^6$ für Wasserstoff, Halogen-$C_1$-$C_8$-Alkyl, wobei Halogen insbesondere für Fluor oder Chlor steht, oder für $C_1$-$C_8$-Alkyl, welches gegebenenfalls durch $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, $C_1$-$C_4$-Alkoxy-carbonyl, $C_1$-$C_4$-Alkylamino-carbonyl, Di-($C_1$-$C_4$-alkyl)-aminocarbonyl, N-($C_1$-$C_4$-alkyl)-phenylamino-carbonyl oder Cyano substituiert ist, $C_2$-$C_6$-Alkenyl, Halogen-$C_2$-$C_6$-alkenyl, $C_3$-$C_6$-Alkinyl, $C_3$-$C_6$-Cycloalkyl, $C_3$-$C_6$-Cycloalkyl-$C_1$-$C_2$-alkyl, für Benzyl, Phenylethyl, Benzhydryl oder Phenyl, welche jeweils gegebenenfalls in der aromatischen Komponente durch Fluor, Chlor, Brom, Cyano, Nitro, $C_1$-$C_4$-Alkyl, $C_1$-$C_4$-Alkoxy und/oder $C_1$-$C_4$-Alkoxy-carbonyl substituiert sind, steht,

$R^7$ für Wasserstoff oder $C_1$-$C_4$-Alkyl steht und

$R^8$ für Wasserstoff, Halogen-$C_1$-$C_4$-alkyl, wobei Halogen, insbesondere für Fluor oder Chlor steht, oder für $C_1$-$C_4$-Alkyl, welches gegebenenfalls durch Cyano, Nitro, $C_1$-$C_4$-Alkoxy oder $C_1$-$C_4$-Alkoxy-carbonyl substituiert ist, $C_3$-$C_6$-Alkenyl, $C_3$-$C_6$-Alkinyl, $C_3$-$C_6$-Cycloalkyl, für Benzyl, Phenylethyl, Benzylhydryl oder Phenyl, welche jeweils in der aromatischen Komponente gegebenenfalls durch Fluor, Chlor, Brom, Cyano, Nitro, $C_1$-$C_4$-Alkyl, Trifluormethyl, $C_1$-$C_4$-Alkoxy, Difluormethoxy, Trifluormethoxy, $C_1$-$C_4$-Alkylthio, Trifluormethylthio oder $C_1$-$C_4$-Alkoxy-carbonyl substituiert sind, $C_1$-$C_4$-Alkyl-carbonyl, $C_1$-$C_4$-Alkoxy-carbonyl, $C_1$-$C_4$-Alkylsulfonyl, Phenylsulfonyl oder Naphthylsulfonyl, wobei Phenylsulfonyl oder Naphthylsulfonyl gegebenenfalls durch Fluor, Chlor, Brom, Cyano, Nitro, $C_1$-$C_4$-Alkyl, Trifluormethyl, $C_1$-$C_4$-Alkoxy, Difluormethoxy, Trifluormethoxy, $C_1$-$C_4$-Alkylthio, Trifluormethylthio und/oder $C_1$-$C_4$-Alkoxy-carbonyl substituiert sind, oder für Pyrimidinyl steht.

Die Erfindung betrifft insbesondere Verbindungen der Formel (I), in welcher

A für Methylen ($-CH_2-$), Dimethylen ($-CH_2CH_2-$), Trimethylen ($-CH_2CH_2CH_2-$), Ethyliden

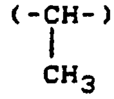

oder Propyliden

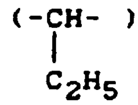

steht,

R für Methyl, Ethyl, Propyl, Isopropyl, Butyl, Isobutyl, sec-Butyl oder tert-Butyl steht,

$R^1$ für Cyano, Fluor oder Chlor steht,

$R^2$ für Wasserstoff, Fluor oder Chlor steht,

$R^3$ für Chlor, Trifluormethyl oder Trifluormethylsulfonyl steht,

$R^4$ für Wasserstoff, Fluor oder Chlor steht,

X für Stickstoff oder die Gruppierung C-$R^5$ steht, worin

$R^5$ für Wasserstoff, Fluor oder Chlor steht,

Y für Sauerstoff oder Schwefel steht, und

Z für Sauerstoff, die Gruppierung N-$(O)_n$-$R^6$ oder

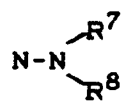

steht, worin

n für die Zahlen 0 oder 1 steht und

$R^6$ für Wasserstoff, $C_1$-$C_4$-Alkyl, $C_3$-$C_4$-Alkenyl, $C_1$-$C_3$-Alkoxy-carbonyl-$C_1$-$C_2$-alkyl, $C_1$-$C_3$-Alkylamino-carbonyl-$C_1$-$C_2$-alkyl, Di-($C_1$-$C_3$-alkyl)-amino-carbonyl-$C_1$-$C_2$-alkyl, N-($C_1$-$C_3$-Alkyl)-phenylamino-carbonyl-$C_1$-$C_2$-alkyl oder Benzyl, welches gegebenenfalls in der aromatischen Komponente durch Fluor, Chlor, Methyl, Methoxy und/oder Methoxycarbonyl substituiert ist, steht,

$R^7$ für Wasserstoff oder Methyl steht und

$R^8$ für $C_1$-$C_4$-Alkyl, Phenyl, welches gegebenenfalls durch Fluor, Chlor, Brom, Methyl, Ethyl, Trifluormethyl, Methoxy, Ethoxy, Difluormethoxy und/oder Trifluormethoxy substituiert ist, Acetyl, Propionyl, Methoxycarbonyl, Ethoxycarbonyl, Methylsulfonyl, Ethylsulfonyl, Phenylsulfonyl oder Tolylsulfonyl steht.

Ganz besonders bevorzugte Gruppen von Verbindungen der Formel (I) sind diejenigen der nachstehenden Formeln (IA) bis (IE), in welchen jeweils A, R, $R^1$, $R^2$, $R^3$, $R^4$, X, Y und Z die oben als insbesondere bevorzugt angegebenen Bedeutungen haben.

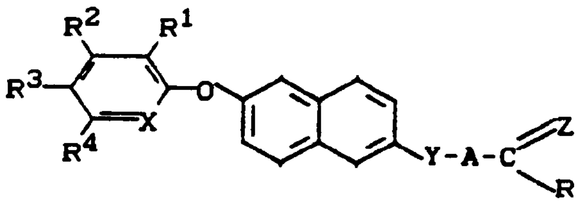

(IA)

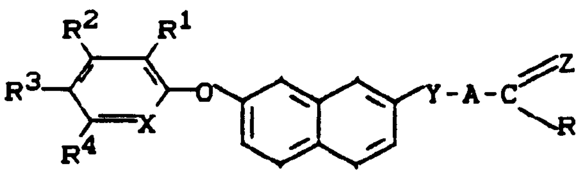

(IB)

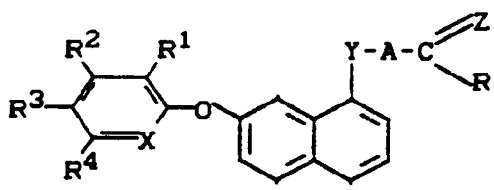

(IC)

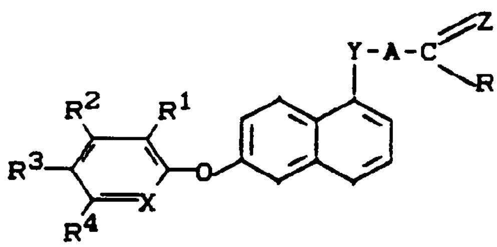

(ID)

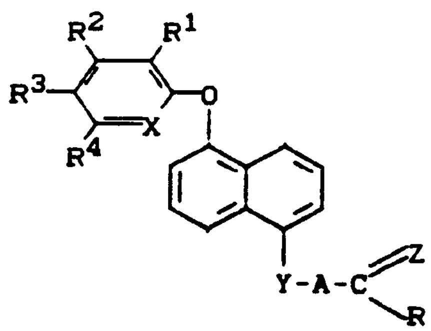

(IE)

Verwendet man für das erfindungsgemäße Verfahren (a) 7-(3-Chlor-5-trifluormethyl-pyridin-2-yl-oxy)-naphthalin-1-yl-mercaptan und Chlormethyl-tert-butylketon als Ausgangsstoffe, so kann der Reaktionsablauf durch das folgende Formelschema wiedergegeben werden:

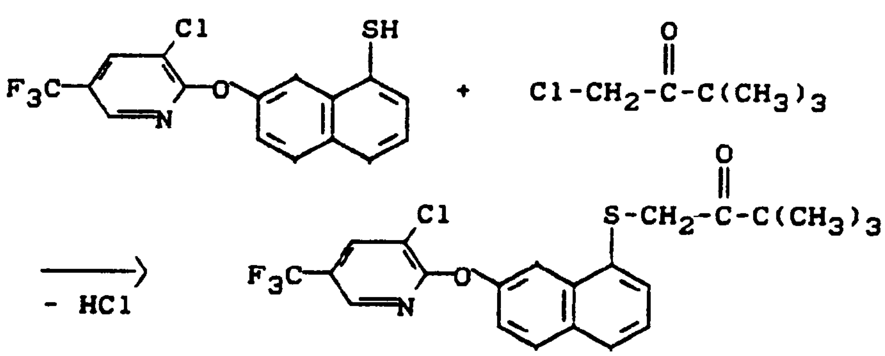


Verwendet man für das erfindungsgemäße Verfahren (b) (7-(2,6-Dichlor-4-trifluormethyl-phenoxy)-naphthalin-2-yl-oxy-methyl)-methylketon und Methansulfonsäurehydrazid als Ausgangsstoffe, so kann der Reaktionsablauf durch das folgende Formelschema wiedergegeben werden:

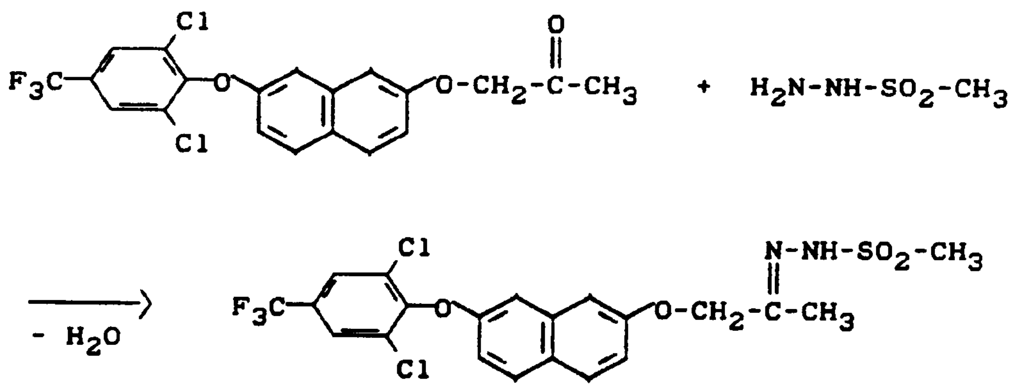


Verwendet man für das erfindungsgemäße Verfahren (c) 5-(3,5-Dichlor-pyridin-2-yl-oxy)-1-(3-hydroximino-butan-2-yl-oxy)-naphthalin und Bromessigsäurebutylester als Ausgangsstoffe, so kann der Reaktionsablauf durch das folgende Formelschema wiedergegeben werden:

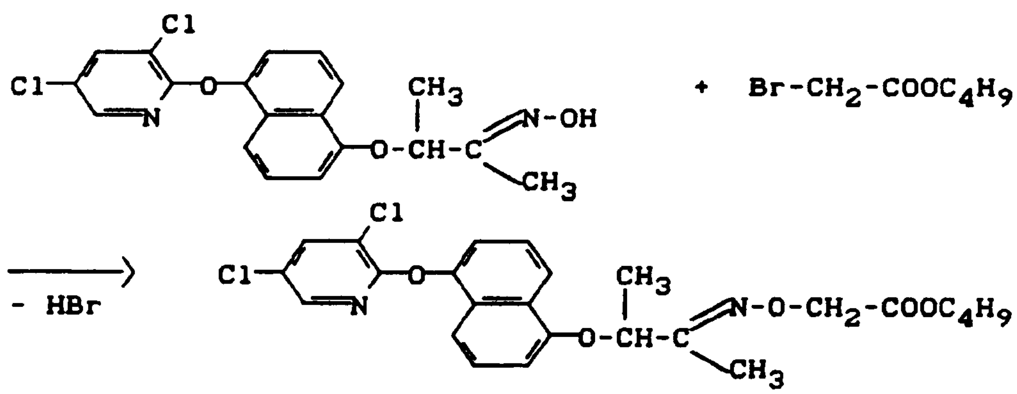


Die beim erfindungsgemäßen Verfahren (a) zur Herstellung von Verbindungen der Formel (I) als Ausgangstoffe zu verwendenden Hydroxy- bzw. Mercaptonaphthaline sind durch die Formel (II) allgemein definiert.

In Formel (II) haben $R^1$, $R^2$, $R^3$, $R^4$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, X und Y angegeben wurden.

Beispiele für die Ausgangsstoffe der Formel (II) sind in der nachstehenden Tabelle 1 aufgeführt.

Die in Tabelle 1 angegebenen Beispiele gelten im einzelnen jeweils für die ganz besonders bevorzugten Gruppen von Verbindungen der Formel (I), welche oben durch die Formeln (IA), (IB), (IC), (ID) und (IE) skizziert sind.

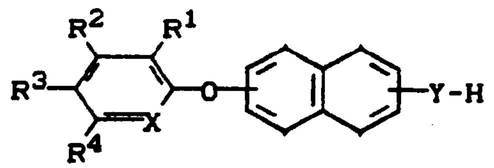

(II)

Tabelle 1: Beispiele für die Ausgangsstoffe der Formel (II)

| $R^1$ | $R^2$ | $R^3$ | $R^4$ | X | Y |
|---|---|---|---|---|---|
| Cl | H | Cl | H | CH | O |
| Cl | H | Cl | H | CH | S |
| Cl | H | Cl | H | N | O |
| Cl | H | Cl | H | N | S |
| Cl | H | $CF_3$ | H | CH | O |
| Cl | H | $CF_3$ | H | CH | S |
| Cl | H | $CF_3$ | H | N | O |
| Cl | H | $CF_3$ | H | N | S |
| Cl | H | $CF_3$ | H | C-Cl | O |
| Cl | H | $CF_3$ | H | C-Cl | S |
| Cl | H | $CF_3$ | H | C-F | O |
| Cl | H | $CF_3$ | H | C-F | S |
| Cl | H | $CF_3$ | F | C-Cl | O |
| Cl | H | $CF_3$ | F | C-Cl | S |
| Cl | H | $CF_3$ | Cl | C-Cl | O |
| Cl | H | $CF_3$ | Cl | C-Cl | S |
| CN | H | $CF_3$ | H | CH | O |
| Cl | H | $SO_2CF_3$ | H | CH | O |
| Cl | H | $SO_3CF_3$ | H | C-Cl | O |
| F | H | $CF_3$ | H | C-F | O |

Die Ausgangsstoffe der Formel (II) sind bekannt oder Gegenstand vorgängiger, nicht vorveröffentlichter Patentanmeldungen (vgl. EP-A 179 015, DE-A 37 31 801, DE-A 37 33 067, DE-P 37 37 179 vom 03. November 1987 und DE-P 3 823 318 vom 09. Juli 1988).

Man erhält die Verbindungen der Formel (II) wenn man

(α) für den Fall, daß Y für Sauerstoff steht, Halogen-(hetero)arylverbindungen der allgemeinen Formel (VII)

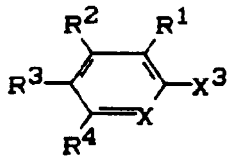

(VII)

in welcher
$R^1$, $R^2$, $R^3$, $R^4$ und X die oben angegebenen Bedeutungen haben und
$X^3$ für Fluor oder Chlor steht,
mit Dihydroxynaphthalinen der allgemeinen Formel (VIII)

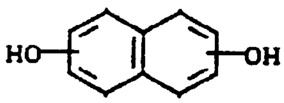

(VIII)

in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20 °C und 150 °C umsetzt und nach üblichen Methoden aufarbeitet, oder

(β) für den Fall, daß Y für Schwefel steht, (Hetero)-Aryloxynaphthalinsulfonsäurechloride der allgemeinen Formel (IX)

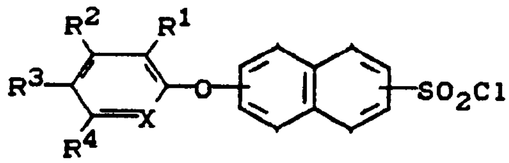

(IX)

in welcher
$R^1$, $R^2$, $R^3$, $R^4$ und X die oben angegebenen Bedeutungen haben,
mit Reduktionsmitteln, wie z. B. Zinkstaub, gegebenenfalls in Gegenwart organischer Lösungsmittel, wie z. B. Tetrahydrofuran oder Dioxan, und gegebenenfalls in Gegenwart wäßriger Mineralsäuren, wie z. B. Salzsäure oder Schwefelsäure, bei Temperaturen zwischen 0 °C und 150 °C umsetzt und nach üblichen Methoden aufarbeitet.

Die als Ausgangsstoffe benötigten Halogen-(hetero)aryl-Verbindungen sind durch die Formel (VII) allgemein definiert. In Formel (VII) haben $R^1$, $R^2$, $R^3$, $R^4$ und X vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden.

Als Beispiele für die Halogen-(hetero)aryl-Verbindungen der Formel (VII) seien genannt:
4-Chlor-benzotrifluorid, 3,4-Dichlor-benzotrifluorid, 3,4,5-Trichlor-benzotrifluorid, 3,4-Dichlor-5-fluorbenzotrifluorid, 2,3,4,5-Tetrachlor-benzotrifluorid, 3,5-Dichlor-2,4-difluor-benzotrifluorid, 3-Chlor-4,5-difluorbenzotrifluorid sowie 2,3,5-Trichlor-pyridin und 2,3-Dichlor-5-trifluormethyl-pyridin.

Die Verbindungen der Formel (VII) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x)).

Als Beispiele für die weiter als Ausgangsstoffe benötigten Dihydroxynaphthaline der Formel (VIII) seien genannt:
1,5-Dihydroxy-naphthalin, 1,6-Dihydroxy-naphthalin, 1,7-Dihydroxy-naphthalin, 2,6-Dihydroxy-naphthalin und 2,7-Dihydroxy-naphthalin.

Die Verbindungen der Formel (VIII) sind bekannte organische Synthesechemikalien.

Die weiter als Ausgangsstoffe benötigten (Hetero)Aryloxynaphthalinsulfonsäurechloride sind durch die Formel (IX) allgemein definiert.

In Formel (IX) haben $R^1$, $R^2$, $R^3$, $R^4$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden.

Als Beispiele für die Ausgangsstoffe der Formel (IX) seien genannt:
5-, 6- und 7-(3,5-Dichlor-pyridin-2-yl-oxy)-, -(2-Chlor-4-trifluormethyl-phenoxy)-, -(2,6-Dichlor-4-trifluormethyl-phenoxy)-, -(2-Chlor-6-fluor-4-trifluormethyl-phenoxy)-, -(2,3,6-Trichlor-4-trifluormethyl-phenoxy)- und (2,6-Dichlor-3-fluor-4-trifluormethyl-phenoxy)-naphthalin-1-sulfonsäurechlorid sowie 6- und 7-(3,5-Dichlorpyridin-2-yl-oxy)-, -(2-Chlor-4-trifluormethyl-phenoxy)-(2,6-Dichlor-4-trifluormethyl-phenoxy)-, -(2-Chlor-6-fluor-4-trifluormethyl-phenoxy)-, -(2,3,6-Trichlor-4-trifluormethyl-phenoxy)- und -(2,6-Dichlor-3-fluor-4-trifluormethyl-phenoxy)-naphthalin-2-sulfonsäurechlorid.

Die Verbindungen der Formel (IX) sind Gegenstand einer nicht vorveröffentlichten Patentanmeldung (vgl. DE-P 3 823 318 vom 09. Juli 1988).

Man erhält die (Hetero)Aryloxynaphthalinsulfonsäurechloride der Formel (IX), wenn man Halogen(hetero)arylverbindungen der Formel (VIII) mit Hydroxynaphthalinsulfonsäuren der allgemeinen Formel (X)

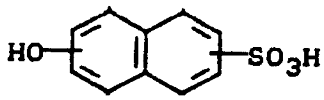
(X)

oder deren Metallsalzen

gegebenenfalls in Gegenwart eines Säureakzeptors, wie z. B. Kaliumhydroxid oder Natriumhydroxid, und gegebenenfalls in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20 °C und 150 °C umsetzt, die hierbei gebildeten (Hetero)Aryloxynaphthalinsulfonsäuren der allgemeinen Formel (XI)

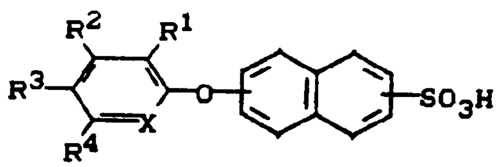
(XI)

in welcher

$R^1$, $R^2$, $R^3$, $R^4$ und X die oben angegebenen Bedeutungen haben,

bzw. deren Metallsalze

nach üblichen Methoden isoliert, mit einem Halogenierungsmittel, wie z. B. Thionylchlorid, Phosgen, Phosphor(V)-chlorid und/oder Phosphorylchlorid, gegebenenfalls in Gegenwart eines Verdünnungsmittels, wie z. B. Methylenchlorid, Chloroform, Tetrachlormethan, Chlorbenzol oder o-Dichlorbenzol, bei Temperaturen zwischen 0 °C und 100 °C umsetzt und nach üblichen Methoden aufarbeitet.

Als Beispiele für die als Ausgangsstoffe benötigten Hydroxynaphthalinsulfonsäuren der Formel (X) und ihre Salze seien genannt:
6-Hydroxy-naphthalin-2-sulfonsäure sowie deren Natrium- und Kalium-Salze, 7-Hydroxy-naphthalin-2-sulfonsäure sowie deren Natrium- und Kalium-Salze, 7-Hydroxy-naphthalin-1-sulfonsäure sowie deren Natrium- und Kalium-Salze, 6-Hydroxy-naphthalin-1-sulfonsäure sowie deren Natrium- und Kalium-Salze und 5-Hydroxy-naphthalin-1-sulfonsäure sowie deren Natrium- und Kalium-Salze.

Die Hydroxynaphthalinsulfonsäuren der Formel (X) sind bekannte organische Synthesechemikalien.

Die beim erfindungsgemäßen Verfahren (a) zur Herstellung von Verbindungen der Formel (I) weiter als Ausgangsstoffe zu verwendenden Alkylierungsmittel sind durch die Formel (III) allgemein definiert.

In Formel (III) haben A, R und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R und Z angegeben wurden und $X^1$ steht vorzugsweise für Chlor oder Brom.

Beispiele für die Ausgangsstoffe der Formel (III) sind in der nachstehenden Tabelle 2 aufgeführt.

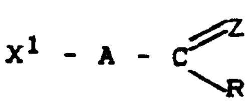
(III)

Tabelle 2: Beispiele für die Ausgangsstoffe der Formel (III)

| $X^1$ | A | R | Z |
|---|---|---|---|
| Cl | $CH_2$ | $CH_3$ | O |
| Br | $CH_2$ | $CH_3$ | O |
| Cl | $CH_2$ | $C_2H_5$ | O |
| Cl | $CH_2$ | $C_3H_7$ | O |
| Cl | $CH_2$ | $CH(CH_3)_2$ | O |
| Cl | $CH_2$ | $C_4H_9$ | O |
| Cl | $CH_2$ | $C(CH_3)_3$ | O |
| Cl | $CH_2CH_2$ | $CH_3$ | O |
| Cl | $CH_2CH_2$ | $C(CH_3)_3$ | O |
| Cl | $CHCH_3$ | $CH_3$ | O |
| Cl | $CHCH_3$ | $C_2H_5$ | O |
| Cl | $CH_2$ | $CH_3$ | $NCH_3$ |

Tabelle 2 - Fortsetzung

| $X^1$ | A | R | Z |
|---|---|---|---|
| Cl | $CH_2$ | $CH_3$ | $NC_2H_5$ |
| Cl | $CH_2$ | $CH_3$ | $NC_3H_7$ |
| Cl | $CH_2$ | $CH_3$ | $NCH(CH_3)_2$ |
| Cl | $CH_2$ | $CH_3$ | $NC_4H_9$ |
| Cl | $CH_2$ | $CH_3$ | $NOCH_3$ |
| Cl | $CH_2$ | $CH_3$ | $NOC_2H_5$ |
| Cl | $CH_2$ | $CH_3$ | $NOC_3H_7$ |
| Cl | $CH_2$ | $CH_3$ | $NOCH(CH_3)_2$ |
| Cl | $CH_2$ | $CH_3$ | $NOC_4H_9$ |
| Cl | $CH_2$ | $CH_3$ | $NOCH_2CH(CH_3)_2$ |
| Cl | $CH_2$ | $CH_3$ | $NN(CH_3)_2$ |
| Cl | $CH_2$ | $CH_3$ | $NNHCH_3$ |
| Cl | $CH_2$ | $CH_3$ | $NNHC_2H_5$ |
| Cl | $CH_2$ | $CH_3$ | $NNHC_3H_7$ |
| Cl | $CH_2$ | $CH_3$ | $NNHCH(CH_3)_2$ |
| Cl | $CH_2$ | $CH_3$ | $NNHC_4H_9$ |
| Cl | $CH_2$ | $CH_3$ | $NNHC(CH_3)_3$ |
| Cl | $CH_2$ | $CH_3$ | $NCH_2COOCH_3$ |
| Cl | $CH_2$ | $CH_3$ | $NCH_2COOC_2H_5$ |
| Cl | $CH_2$ | $CH_3$ | $NCH(CH_3)COOCH_3$ |
| Cl | $CH_2$ | $CH_3$ | $NCH(CH_3)COOC_2H_5$ |

Die Ausgangsstoffe der Formel (III) sind bekannt und/oder können nach an sich bekannten Verfahren hergestellt werden (vgl. Org. Prep. Proced. Int. 11 (1979), 115 ff.; loc. cit. 12 (1980), 49 ff. Synthesis 1982, 43 ff.).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Aprotische polare organische Lösungsmittel, wie z.B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als Ausgangsstoffe zu verwendenden Carbonylverbindungen sind durch die Formel (Ia) allgemein definiert.

In Formel (Ia) haben A, R, $R^1$, $R^2$, $R^3$, $R^4$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, $R^1$, $R^2$, $R^3$, $R^4$, X und Y angegeben wurden.

Beispiele für die Ausgangsstoffe der Formel (Ia) sind in der nachstehenden Tabelle 3 aufgeführt.

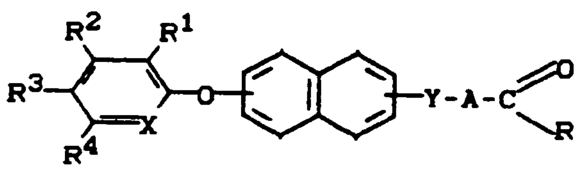

(Ia)

Tabelle 3: Beispiele für die Ausgangsstoffe der Formel (Ia)

| $R^1$ | $R^2$ | $R^3$ | $R^4$ | X | Y | A | R |
|---|---|---|---|---|---|---|---|
| Cl | H | Cl | H | CH | O | $CH_2$ | $CH_3$ |
| Cl | H | Cl | H | CH | S | $CH_2$ | $CH_3$ |
| Cl | H | Cl | H | N | O | $CH_2$ | $CH_3$ |
| Cl | H | Cl | H | N | S | $CH_2$ | $CH_3$ |
| Cl | H | $CF_3$ | H | CH | O | $CH_2$ | $CH_3$ |
| Cl | H | $CF_3$ | H | CH | S | $CH_2$ | $CH_3$ |
| Cl | H | $CF_3$ | H | N | O | $CH_2$ | $CH_3$ |
| Cl | H | $CF_3$ | H | N | S | $CH_2$ | $CH_3$ |
| Cl | H | $CF_3$ | H | C-Cl | O | $CH_2$ | $CH_3$ |
| Cl | H | $CF_3$ | H | C-Cl | S | $CH_2$ | $CH_3$ |
| Cl | H | $CF_3$ | H | C-F | O | $CH_2$ | $CH_3$ |
| Cl | H | $CF_3$ | H | C-F | S | $CH_2$ | $CH_3$ |
| Cl | H | $CF_3$ | F | C-Cl | O | $CH_2$ | $CH_3$ |
| Cl | H | $CF_3$ | F | C-Cl | S | $CH_2$ | $CH_3$ |
| Cl | H | $CF_3$ | Cl | C-Cl | S | $CH_2$ | $CH_3$ |
| Cl | H | $CF_3$ | Cl | C-Cl | O | $CH_2$ | $CH_3$ |
| CN | H | $CF_3$ | H | CH | O | $CH_2$ | $CH_3$ |
| Cl | H | $SO_2CF_3$ | H | CH | O | $CH_2$ | $CH_3$ |
| Cl | H | $SO_2CF_3$ | H | C-Cl | O | $CH_2$ | $CH_3$ |
| F | H | $CF_3$ | H | C-F | O | $CH_2$ | $CH_3$ |
| Cl | H | Cl | H | N | O | $CH_2$ | $C_2H_5$ |

Tabelle 3 - Fortsetzung

| $R^1$ | $R^2$ | $R^3$ | $R^4$ | X | Y | A | R |
|---|---|---|---|---|---|---|---|
| Cl | H | $CF_3$ | H | C-Cl | O | $CH_2$ | $C_2H_5$ |
| Cl | H | $CF_3$ | H | C-F | O | $CH_2$ | $C_2H_5$ |
| Cl | H | $CF_3$ | H | C-F | O | $CH_2$ | $CH(CH_3)_2$ |
| Cl | H | $CF_3$ | H | C-Cl | O | $CH_2$ | $C(CH_3)_3$ |
| Cl | H | $CF_3$ | H | C-F | O | $CH_2$ | $C(CH_3)_3$ |
| Cl | H | $CF_3$ | H | N | O | $CH_2CH_2$ | $CH_3$ |
| Cl | H | $CF_3$ | H | CH | O | $CHCH_3$ | $CH_3$ |
| Cl | H | Cl | H | N | O | $CHCH_3$ | $C_2H_5$ |
| Cl | H | $CF_3$ | H | C-Cl | O | $CH_2$ | $CH_2Cl$ |
| Cl | H | $CF_3$ | H | C-Cl | O | $CH_2$ | $CF_3$ |
| Cl | H | Cl | H | N | O | $CH_2$ | $CF_3$ |
| Cl | H | Cl | H | N | O | $CH_2$ | $CH_2Cl$ |

Die in Tabelle 3 angegebenen Beispiele gelten im einzelnen jeweils für die ganz besonders bevorzugten Gruppen von Verbindungen der Formel (I), welche oben durch die Formeln (IA), (IB), (IC), (ID) und (IE) skizziert sind.

Die Ausgangsstoffe der Formel (Ia) für das erfindungsgemäße Verfahren (b) sind erfindungsgemäße, neue Verbindungen; sie können nach dem erfindungsgemäßen Verfahren (a) hergestellt werden.

Die beim erfindungsgemäßen Verfahren (b) weiter als Ausgangsstoffe zu verwendenden Aminoverbindungen sind durch die Formeln (IV) und (V) allgemein definiert. In den Formeln (IV) und (V) haben n, $R^6$, $R^7$ und $R^8$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^6$, $R^7$ und $R^8$ angegeben wurden.

Als Beispiele für die Ausgangsstoffe der Formeln (IV) und (V) seien genannt:
Ammoniak, Methylamin, Ethylamin, Propylamin, Isopropylamin, Butylamin, Isobutylamin, sec-Butylamin, tert-Butylamin, Allylamin, Aminoessigsäure-methylester und -ethylester, α-Amino-propionsäure-methylester und -ethylester, Benzylamin, 2-Fluor-, 3-Fluor- und 4-Fluor-benzylamin, 2-Chlor-, 3-Chlor- und 4-Chlor-benzylamin, 2-Methyl-, 3-Methyl- und 4-Methyl-benzylamin, 4-Methoxy-benzylamin, 3,4-Dimethyl- und 3,4-Dimethoxy-benzylamin, Hydroxylamin, O-Methyl-, O-Ethyl-, O-Propyl-, O-Isopropyl-, O-Butyl-, O-Isobutyl-, O-sec-Butyl-, O-Allyl-, O-Benzyl-, O-(2-Fluor-benzyl)-, O-(4-Fluor-benzyl)-, O-(2-Chlor-benzyl)-, O-(4-Chlor-benzyl)-, O-(2-Methyl-benzyl)-, O-(3-Methyl-benzyl)-, O-(4-Methyl-benzyl)-, O-(3,4-Dimethyl-benzyl)-, O-(4-Methoxy-benzyl)-, O-(3,4-Dimethoxy-benzyl)-, O-(4-Methoxycarbonyl-benzyl)- und O-(4-Ethoxycarbonyl-benzyl)-hydroxylamin, Aminooxyessigsäure-methylester, -ethylester, -propylester, -isopropylester, -methylamid, -ethylamid, -propylamid, -isopropylamid, -dimethylamid, -diethylamid und -N-methyl-anilid, α-Aminooxy-propionsäure-methylester, -ethylester, -propylester, -isopropylester, -methylamid, -ethylamid, -propylamid, -isopropylamid, -dimethylamid, -diethylamid und -N-methyl-anilid, Hydrazin, Methylhydrazin, N,N-Dimethylhydrazin, Ethylhydrazin, Propylhydrazin, Isopropylhydrazin, Butylhydrazin, Isobutylhydrazin, sec-Butylhydrazin, tert-Butylhydrazin, Phenylhydrazin, 4-Fluor-, 4-Chlor-, 4-Brom-, 4-Methyl-, 4-Trifluor-methyl- und 4-Methoxyphenylhydrazin, Acethydrazid, Hydrazinoameisensäure-methylester und -ethylester, Methansulfonsäurehydrazid, Benzolsulfonsäurehydrazid und p-Toluolsulfonsäurehydrazid.

Die Aminoverbindungen der Formeln (IV) und (V) sind bekannte organische Synthesechemikalien.

Das erfindungsgemäße Verfahren (b) wird vorzugsweise unter Verwendung von Verdünnungsmitteln durchgeführt. Als Verdünnungsmittel kommen mit Ausnahme von Ketonen diejenigen organischen Lösungsmittel in Betracht, die oben bei der Beschreibung des erfindungsgemäßen Verfahrens (a) angegeben wurden. Zur azeotropen Wasserabscheidung geeignete Lösungsmittel, wie z. B. Benzol, Toluol oder Xylol, werden besonders bevorzugt.

Das erfindungsgemäße Verfahren (b) wird vorzugsweise in Gegenwart eines Katalysators durchgeführt. Bevorzugte Katalysatoren sind starke Säuren, wie z. B. Schwefelsäure, Methansulfonsäure, Benzolsulfonsäure oder p-Toluolsulfonsäure, oder mit Säuren aktivierte Ionenaustauscherharze.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Katalysators durchgeführt, und das Reaktionsgemisch wird mehrere Stunden bei der jeweils erforderlichen Temperatur - vorzugsweise unter Erhitzen am Wasserabscheider - gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden Hydroximinoverbindungen sind durch die Formel (Ib) allgemein definiert.

In Formel (Ib) haben A, R, $R^1$, $R^2$, $R^3$, $R^4$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, $R^1$, $R^2$, $R^3$, $R^4$, X und Y angegeben wurden.

Beispiele für die Ausgangsstoffe der Formel (Ib) sind in der nachstehenden Tabelle 4 aufgeführt.

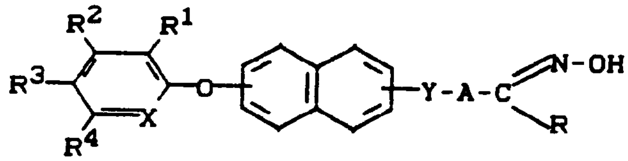

(Ib)

Tabelle 4: Beispiele für die Ausgangsstoffe der Formel (Ib)

| $R^1$ | $R^2$ | $R^3$ | $R^4$ | X | Y | A | R |
|---|---|---|---|---|---|---|---|
| Cl | H | Cl | H | CH | O | $CH_2$ | $CH_3$ |
| Cl | H | Cl | H | CH | S | $CH_2$ | $CH_3$ |
| Cl | H | Cl | H | N | O | $CH_2$ | $CH_3$ |
| Cl | H | Cl | H | N | S | $CH_2$ | $CH_3$ |
| Cl | H | $CF_3$ | H | CH | O | $CH_2$ | $CH_3$ |
| Cl | H | $CF_3$ | H | CH | S | $CH_2$ | $CH_3$ |
| Cl | H | $CF_3$ | H | N | O | $CH_2$ | $CH_3$ |
| Cl | H | $CF_3$ | H | N | S | $CH_2$ | $CH_3$ |
| Cl | H | $CF_3$ | H | C-Cl | O | $CH_2$ | $CH_3$ |
| Cl | H | $CF_3$ | H | C-Cl | S | $CH_2$ | $CH_3$ |
| Cl | H | $CF_3$ | H | C-F | O | $CH_2$ | $CH_3$ |
| Cl | H | $CF_3$ | H | C-F | S | $CH_2$ | $CH_3$ |
| Cl | H | $CF_3$ | F | C-Cl | O | $CH_2$ | $CH_3$ |
| Cl | H | $CF_3$ | F | C-Cl | S | $CH_2$ | $CH_3$ |
| Cl | H | $CF_3$ | Cl | C-Cl | S | $CH_2$ | $CH_3$ |
| Cl | H | $CF_3$ | Cl | C-Cl | O | $CH_2$ | $CH_3$ |
| CN | H | $CF_3$ | H | CH | O | $CH_2$ | $CH_3$ |
| Cl | H | $SO_2CF_3$ | H | CH | O | $CH_2$ | $CH_3$ |
| Cl | H | $SO_2CF_3$ | H | C-Cl | O | $CH_2$ | $CH_3$ |
| F | H | $CF_3$ | H | C-F | O | $CH_2$ | $CH_3$ |
| Cl | H | Cl | H | N | O | $CH_2$ | $C_2H_5$ |

Tabelle 4 - Fortsetzung

| $R^1$ | $R^2$ | $R^3$ | $R^4$ | X | Y | A | R |
|---|---|---|---|---|---|---|---|
| Cl | H | $CF_3$ | H | C-Cl | O | $CH_2$ | $C_2H_5$ |
| Cl | H | $CF_3$ | H | C-F | O | $CH_2$ | $C_2H_5$ |
| Cl | H | $CF_3$ | H | C-F | O | $CH_2$ | $CH(CH_3)_2$ |
| Cl | H | $CF_3$ | H | C-Cl | O | $CH_2$ | $C(CH_3)_3$ |
| Cl | H | $CF_3$ | H | C-F | O | $CH_2$ | $C(CH_3)_3$ |
| Cl | H | $CF_3$ | H | N | O | $CH_2CH_2$ | $CH_3$ |
| Cl | H | $CF_3$ | H | CH | O | $CHCH_3$ | $CH_3$ |
| Cl | H | Cl | H | N | O | $CHCH_3$ | $C_2H_5$ |
| Cl | H | $CF_3$ | H | C-Cl | O | $CH_2$ | $CH_2Cl$ |
| Cl | H | $CF_3$ | H | C-Cl | O | $CH_2$ | $CF_3$ |
| Cl | H | Cl | H | N | O | $CH_2$ | $CF_3$ |
| Cl | H | Cl | H | N | O | $CH_2$ | $CH_2Cl$ |

Die in Tabelle 4 angegebenen Beispiele gelten im einzelnen jeweils für die ganz besonders bevorzugten Gruppen von Verbindungen der Formel (I), welche oben durch die Formeln (IA), (IB), (IC), (ID) und (IE) skizziert sind.

Die Ausgangsstoffe der Formel (Ib) für das erfindungsgemäße Verfahren (c) sind erfindungsgemäße, neue Verbindungen; sie können nach den erfindungsgemäßen Verfahren (a) und (b) hergestellt werden.

Die beim erfindungsgemäßen Verfahren (c) zur Herstellung von Verbindungen der Formel (I) weiter als Ausgangsstoffe zu verwendenden Alkylierungsmittel sind durch die Formel (VI) allgemein definiert.

In Formel (VI) hat $R^6$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^6$ angegeben wurde und $X^2$ steht vorzugsweise für Chlor, Brom, Iod, Methylsulfonyl, Phenylsulfonyl oder p-Toluolsulfonyl.

Als Beispiele für die Ausgangsstoffe der Formel (VI) seien genannt:

Chlor-, Brom- oder Iod-methan, -ethan, -propan, -butan, -essigsäure-methylester, -essigsäure-ethylester, -essigsäure-dimethylamid, -essigsäure-diethylamid, -essigsäure-N-methyl-anilid, Allylbromid, Benzyl-, 2-Fluorbenzyl-, 4-Fluor-benzyl-, 4-Chlor-benzyl-, 4-Methyl-benzyl- und 4-Methoxy-benzyl-chlorid, 2-Brom-propan, α-Brom- und α-Chlor-propionsäure-methylester, -propionsäure-ethylester, -propionsäure-methylamid, -propionsäure-ethylamid, -propionsäure-dimethylamid, -propionsäure-diethylamid und -propionsäure-N-methyl-anilid.

Die Ausgangsstoffe der Formel (VI) sind bekannte organische Synthesechemikalien.

Das erfindungsgemäße Verfahren (c) wird vorzugsweise unter Verwendung von Verdünnungsmitteln durchgeführt. Als Verdünnungsmittel kommen vor allem diejenigen organischen Lösungsmittel in Betracht, die oben bei der Beschreibung des erfindungsgemäßen Verfahrens (a) angegeben wurden.

Das erfindungsgemäße Verfahren (c) wird vorzugsweise in Gegenwart eines Säureakzeptors durchgeführt. Als solche kommen vor allem diejenigen Säurebindemittel in Betracht, die oben bei der Beschreibung des erfindungsgemäßen Verfahrens (a) angegeben wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Umkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen, wie Getreide, Mais, Reis und Soja, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 2-Chlor-4-ethylamino-6-(3-cyanopropylamino)-1,3,5-triazin (CYANAZIN); 2,6-Dichlorbenzonitril (DICHLOBENIL); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on [DIMETHAZONE); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N′-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); N-Phosphonomethyl-glycin (GLYPHOSATE); 2-{4-[(3-Chlor-5-(trifluormethyl)-2-pyridinyl)-oxy]-phenoxy}-propansäure bzw. deren Ethylester (HALOXYFOP); Methyl-2-[4,5-dihydro-4-methyl-4-(1-methylethyl)-5-oxo-1H-imidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxy-benzonitril (IOXYNIL); N,N-Dimethyl-N′-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP; 2-{[[((4-Methoxy-6-methyl-1,3,5-triazin-2-yl)-amino)-carbonyl]-amino]-sulfonyl}-benzoesäure oder deren Methylester (METSULFURON); (2-Chlor-4-trifluormethylphenyl)-(3-ethoxy-4-nitro-phenyl)-ether (OXYFLUORFEN); 0-(6-Chlor-3-phenyl-pyridazin-4-yl)-S-octyl-thiocarbonat (PYRIDATE); 2-Chlor-4,6-bis-(ethylamino)-1,3,5-triazin (SIMAZIN); 2,4-Bis-[N-ethylamino]-6-methylthio-1,3,5-triazin (SIMETRYNE); Methyl-2-{[(4,6-dimethyl-2-pyrimidinyl)-aminocarbonyl]-aminosulfonyl}-benzoat (SULFOMETURON); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

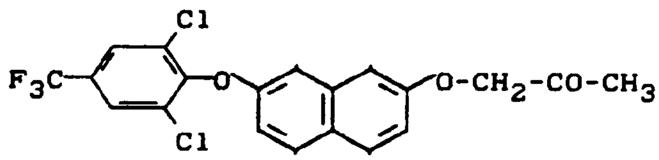

(Verfahren (a))

Eine Mischung aus 1,9 g (5,0 Mol) 7-(2,6-Dichlor-4-trifluormethyl-phenoxy)-2-naphthol, 0,5 g (6,0 mMol) Chloraceton, 0,8 g (6,0 mMol) Kaliumcarbonat und 50 ml Acetonitril wird 5 Stunden unter Rückfluß zum Sieden erhitzt und dann 15 Stunden bei 20 °C gerührt. Anschließend wird mit Wasser auf etwa das doppelte Volumen verdünnt und das hierbei kristallin angefallene Produkt durch Absaugen isoliert.

Man erhält 2,0 g (93 % der Theorie) (7-(2,6-Dichlor-4-trifluormethyl-phenoxy)-naphthalin-2-yl-oxy)-aceton vom Schmelzpunkt 132°C.

Beispiel 2

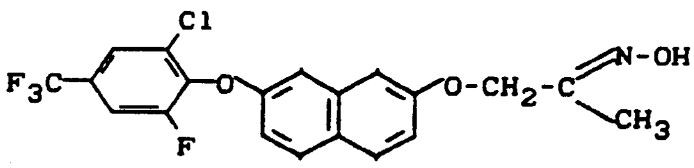

(Verfahren (b))

Eine Mischung aus 24,8 g (0,06 Mol) (7-(2-Chlor-6-fluor-4-trifluormethyl-phenoxy)-naphthalin-2-yl-oxy)-aceton, 4,8 g (0,06 Mol) Hydroxylamin-Hydrochlorid, 6,0 g (0,06 Mol) Triethylamin und 500 ml Toluol wird 15 Stunden unter Rückfluß zum Sieden erhitzt. Nach Abkühlen auf 20 °C wird mit Wasser gewaschen, die organische Phase mit Natriumsulfat getrocknet und filtriert. Das Filtrat wird eingeengt, das im Rückstand verbleibende Produkt durch Verrühren mit Ethanol/Hexan zur Kristallisation gebracht und durch Absaugen isoliert.

Man erhält 18,4 g (72 % der Theorie) (7-(2-Chlor-6-fluor-4-trifluormethyl-phenoxy)-naphthalin-2-yl-oxy)-acetonoxim (E/Z = 7/3) vom Schmelzpunkt 153 °C.

Beispiel 3

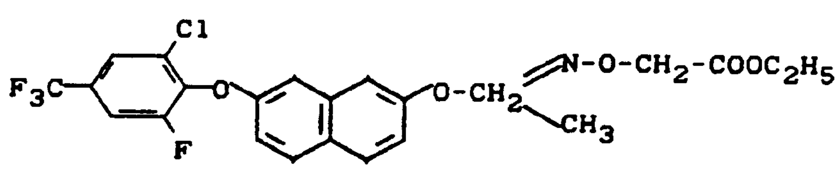

(Verfahren (c))

Eine Mischung aus 8,6 g (0,02 Mol) (7-(2-Chlor-6-fluor-4-trifluormethyl-phenoxy)-naphthalin-2-yl-oxy)-acetonoxim (E/Z = 7/3), 3,5 g (0,021 Mol) Bromessigsäureethylester, 3,2 g (0,023 Mol) Kaliumcarbonat und 100 ml Acetonitril wird 20 Stunden unter Rückfluß zum Sieden erhitzt und anschließend eingeengt. Der Rückstand wird in Essigsäureethylester aufgenommen, mit Wasser gewaschen, mit Natriumsulfat getrocknet und filtriert. Das Filtrat wird eingeengt, der Rückstand mit Methanol verrührt und das hierei kristallin angefallene Produkt durch Absaugen isoliert.

Man erhält 9,6 g (93 % der Theorie) 7-(2-Chlor-6-fluor-3-trifluormethyl-phenoxy)-2-(2-ethoxycarbonylmethoximino-propoxy)-naphthalin (E/Z = 7/3) vom Schmelzpunkt 109 °C.

Analog zu den Beispielen 1 bis 3 sowie entsprechend der allgemeinen Beschreibung der erfindungsgemäßen Herstellungsverfahren können die in der nachstehenden Tabelle 5 aufgeführten Verbindungen der Formel (I) - genauer spezifiziert durch die "Isomerengruppen" der Formeln (IA), (IB), (IC), (ID) und (IE) - hergestellt werden.

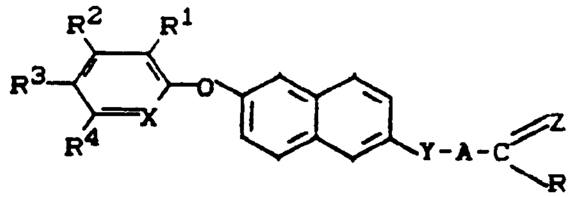

(IA)

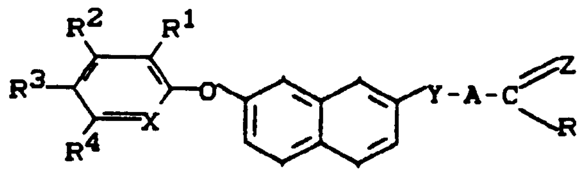

(IB)

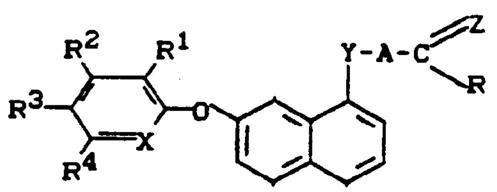

(IC)

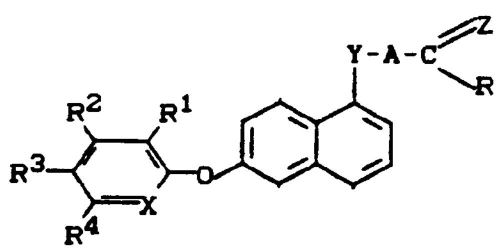

(ID)

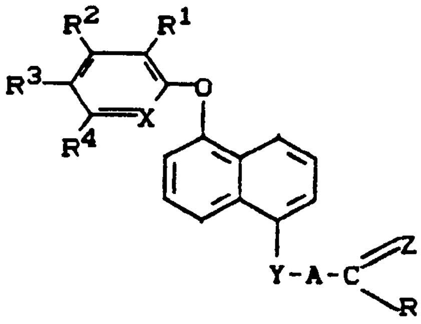

(IE)

**Tabelle 5**: Beispiele für die Verbindungen der Formel (I)

| Bsp.-Nr. | Isomeren-gruppe | $R^1$ | $R^2$ | $R^3$ | $R^4$ | X | Y | A | R | Z | Physikalische Daten |
|---|---|---|---|---|---|---|---|---|---|---|---|
| 4 | IA | Cl | H | $CF_3$ | H | C-Cl | O | $CH_2$ | $CH_3$ | O | Fp: 147 °C |
| 5 | IA | Cl | H | $CF_3$ | H | C-Cl | O | $CH_2$ | $CH_3$ | N-OH (E/Z = 4/1) | Fp: 163 °C |
| 6 | IB | Cl | H | $CF_3$ | H | C-Cl | O | $CH_2$ | $CH_3$ | N-OH (E/Z = 1/1) | Fp: 180 °C |
| 7 | IB | Cl | H | $CF_3$ | H | C-Cl | O | $CH_2$ | $CH_3$ | N-OH (E/Z = 99/1) | Fp: 147 °C |
| 8 | IB | Cl | H | $CF_3$ | H | C-Cl | O | $CH_2$ | $CH_3$ | $N-OCH_3$ (E/Z = 3/1) | |
| 9 | IB | Cl | H | $CF_3$ | H | C-Cl | O | $CH_2$ | $CH_3$ | 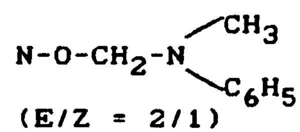 (E/Z = 2/1) | Fp: 114 °C |
| 10 | IA | Cl | H | $CF_3$ | H | C-Cl | O | $CH_2$ | $CH_3$ | $N-O-CH_2-COOC_2H_5$ (E/Z = 7/3) | Fp: 98 °C |
| 11 | IB | Cl | H | $CF_3$ | H | C-Cl | O | $CH_2$ | $CH_3$ | $N-O-CH_2-COOC_2H_5$ (E/Z = 7/3) | Fp: 119 °C |
| 12 | IA | Cl | H | $CF_3$ | H | C-Cl | O | $CH_2$ | $CH_3$ | $N-OCH_3$ (E/Z = 99/1) | Fp: 117 °C |

<u>Tabelle 5</u> - Fortsetzung

| Bsp.-Nr. | Isomerengruppe | $R^1$ | $R^2$ | $R^3$ | $R^4$ | X | Y | A | R | Z | Physikalische Daten |
|---|---|---|---|---|---|---|---|---|---|---|---|
| 13 | IA | Cl | H | $CF_3$ | H | C-Cl | O | $CH_2$ | $CH_3$ | 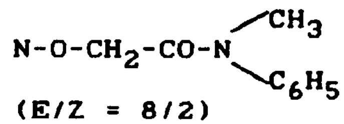 (E/Z = 8/2) | Fp: 165 °C |
| 14 | IB | Cl | H | $CF_3$ | H | C-F | O | $CH_2$ | $CH_3$ | O | Fp: 94 °C |
| 15 | IB | Cl | H | $CF_3$ | H | C-F | O | $CH_2$ | $CH_3$ | 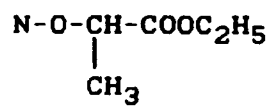 | |
| 16 | IB | Cl | H | $CF_3$ | H | C-Cl | S | $CH_2$ | $CH_3$ | $N-O-CH_2-COOC_2H_5$ | |
| 17 | IB | Cl | H | $CF_3$ | H | C-Cl | O | $CH_2$ | $CH_3$ | $N-CH_2-COOC_2H_5$ | |
| 18 | IB | Cl | H | $CF_3$ | H | C-Cl | O | $CH_2$ | $CH_3$ | $N-O-CH_2-COOCH_3$ | |
| 19 | IB | Cl | H | $CF_3$ | H | C-Cl | S | $CH_2$ | $CH_3$ | $N-O-CH_2-COOCH_3$ | |
| 20 | IB | Cl | H | $CF_3$ | H | C-Cl | O | $CH_2$ | H | $N-O-CH_2-COOC_2H_5$ | |
| 21 | IB | Cl | H | $CF_3$ | H | C-Cl | O | $CH_2$ | H | $N-O-CH_2-COOCH_3$ | |
| 22 | IB | Cl | H | $CF_3$ | H | C-F | S | $CH_2$ | $CH_3$ | $N-O-CH_2-COOCH_3$ | |
| 23 | IB | Cl | H | $CF_3$ | H | C-F | O | $CH_2$ | $CH_3$ | 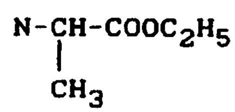 | |

Tabelle 5 - Fortsetzung

| Bsp.-Nr. | Isomerengruppe | $R^1$ | $R^2$ | $R^3$ | $R^4$ | X | Y | A | R | Z | Physikalische Daten |
|---|---|---|---|---|---|---|---|---|---|---|---|
| 24 | IB | Cl | H | $CF_3$ | H | N | O | $CH_2$ | $CH_3$ | $N-CH_2-COOC_3H_7$ | |
| 25 | IB | Cl | H | $CF_3$ | H | N | O | $CH_2$ | $CH_3$ | $N-O-CH_2-COOCH(CH_3)_2$ | |
| 26 | IB | Cl | H | $CF_3$ | H | C-Cl | O | $CH_2$ | $CH_3$ | O | |
| 27 | IB | Cl | H | $CF_3$ | H | C-F | O | $CH_2$ | $CH_3$ | O | |
| 28 | IB | Cl | H | $CF_3$ | H | C-Cl | S | $CH_2$ | $CH_3$ | O | |
| 29 | IA | Cl | H | $CF_3$ | H | C-F | O | $CH_2$ | $CH_3$ | $N-CH_2-COOC_2H_5$ | |
| 30 | IB | Cl | H | $CF_3$ | H | C-F | O | $CH_2$ | $CH_3$ | $N-CH_2-COOC_2H_5$ | |
| 31 | IB | Cl | H | $CF_3$ | H | C-Cl | O | $CH_2$ | $CH_3$ | $N-NH_2$ | Fp: 163° C |
| 32 | IB | Cl | H | $CF_3$ | H | C-Cl | O | $CH_2$ | $CH_3$ | $N-NHCH_2COOC_2H_5$ | (amorph) |
| 33 | IB | Cl | H | $CF_3$ | H | N | O | $CH_2$ | $CH_3$ | O | Fp: 150° C |

<u>Tabelle 5</u> - Fortsetzung

| Bsp.-Nr. | Isomerengruppe | $R^1$ | $R^2$ | $R^3$ | $R^4$ | X | Y | A | R | Z | Physikalische Daten |
|---|---|---|---|---|---|---|---|---|---|---|---|
| 34 | IB | Cl | H | $CF_3$ | H | C-F | S | $CH_2$ | $CH_3$ | O | Fp: 102° C |
| 35 | IB | Cl | H | $CF_3$ | H | N | O | $CH_2$ | $CH_3$ | N-OH | Fp: 128° C |
| 36 | IB | Cl | H | $CF_3$ | H | N | O | $CH_2$ | $CH_3$ | $N-OCH_2COOCH_3$ | |
| 37 | IB | Cl | H | $CF_3$ | H | C-F | S | $CH_2$ | $CH_3$ | N-OH | |
| 38 | IB | Cl | H | $CF_3$ | H | C-F | S | $CH_2$ | $CH_3$ | $NOCH_2COOCH_3$ | |

Ausgangsstoffe der Formel (II)

Beispiel (II-1)

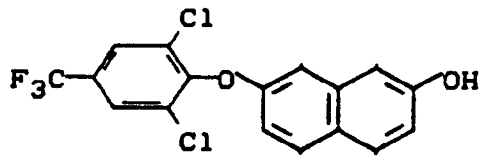

12,5 g (0,05 Mol) 3,4,5-Trichlor-benzotrifluorid werden zu einer auf 60 °C erwärmten Mischung aus 8,0 g (0,05 Mol) 2,7-Dihydroxy-naphthalin, 4,2 g (0,075 Mol) Kaliumhydroxid-Pulver und 100 ml Dimethylsulfoxid langsam unter Rühren gegeben und das Reaktionsgemisch wird dann noch ca. 3 Stunden bei 60 °C gerührt. Nach Abkühlen auf ca. 20 °C wird mit Wasser und Methylenchlorid verdünnt und filtriert. Vom Filtrat wird die organische Phase abgetrennt, mit Wasser gewaschen, mit Natriumsulfat getrocknet und filtriert. Vom Filtrat wird das Lösungsmittel im Wasserstrahlvakuum abdestilliert, der Rückstand mit Petrolether verrührt und das hierbei kristallin angefallene Produkt durch Absaugen isoliert.

Man erhält 4,9 g (26 % der Theorie) 7-(2,6-Dichlor-4-trifluormethyl-phenoxy)-2-naphthol vom Schmelzpunkt 98 °C.

Beispiel (II-2)

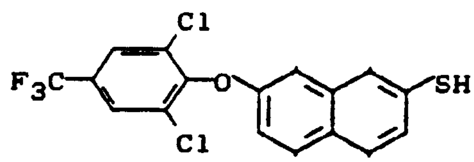

Zu einer Mischung aus 12,5 g (0,19 Mol) Zinkstaub und 100 ml Dioxan werden nacheinander unter Rühren 12,5 g (0,025 Mol) 7-(2,6-Dichlor-4-trifluormethyl-phenoxy)-naphthalin-2-sulfonsäurechlorid und 25 ml konzentrierte Salzsäure gegeben. Das Reaktionsgemisch wird 5 Stunden unter Rückfluß zum Sieden erhitzt und dann weitere 15 Stunden bei 20 °C gerührt. Nach Einengen wird der Rückstand in Methylenchlorid/Wasser aufgenommen, filtriert, das Filtrat geschüttelt, die organische Phase abgetrennt, mit Wasser gewaschen, mit Natriumsulfat getrocknet und filtriert.
Das Filtrat wird eingeengt, der Rückstand mit Ethanol digeriert und das hierbei kristallin angefallene Produkt durch Absaugen isoliert.

Man erhält 4,8 g (49 % Theorie) 7-(2,6-Dichlor-4-trifluormethyl-phenoxy)-naphthalin-2-thiol vom Schmelzpunkt 98°C.

Anwendungsbeispiele

In den folgenden Anwendungsbeispielen wird die nachstehend aufgeführte Verbindung als Vergleichssubstanz herangezogen:

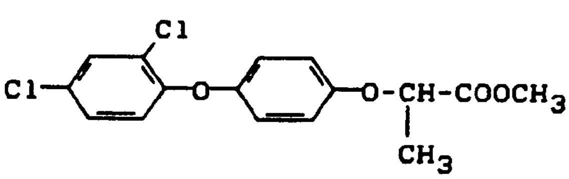

(A)

α-[4-(2,4-Dichlor-phenoxy)-phenoxy]-propionsäure-methylester (Diclofop-methyl)
(bekannt aus DE-OS 22 23 894/Beispiel 86).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen: (11) und (14).

## Patentansprüche

1. Disubstituierte Naphthaline der Formel (I)

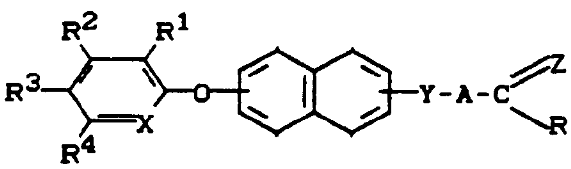

(I)

in welcher
A für gegebenenfalls verzweigtes Alkandiyl steht,
R für Wasserstoff oder für jeweils gegebenenfalls substituiertes Alkyl oder Phenyl steht,
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht,
X für Stickstoff oder die Gruppierung C-$R^5$ steht, worin
$R^5$ für Wasserstoff oder Halogen steht,
Y für Sauerstoff oder Schwefel steht, und
Z für Sauerstoff, die Gruppierung N-$(O)_n$-$R^6$ oder

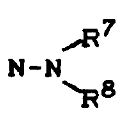

steht, worin
n für die Zahlen 0 oder 1 steht und
$R^6$ für Wasserstoff oder für einen gegebenenfalls substituierten Rest aus der Reihe Alkyl, Alkenyl, Alkinyl, Cycloalkyl, Cycloalkylalkyl, Aralkyl und Aryl steht,
$R^7$ für Wasserstoff oder Alkyl steht und
$R^8$ für Wasserstoff oder einen gegebenenfalls substituierten Rest aus der Reihe Alkyl, Alkenyl, Alkinyl, Cycloalkyl, Aralkyl, Aryl, Alkylcarbonyl, Alkoxycarbonyl, Alkylsulfonyl, Arylsulfonyl und Heteroaryl steht.

2. Disubstituierte Naphthaline der Formel (I) gemäß Anspruch 1,
in welcher
A für gegebenenfalls verzweigtes $C_1$-$C_4$-Alkandiylsteht,
R für Wasserstoff, $C_1$-$C_6$-Alkyl oder Phenyl steht,
$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor, Chlor oder Brom steht,
Y für Sauerstoff oder Schwefel steht, und
Z für Sauerstoff, die Gruppierung N-$(O)_n$-$R^6$ oder

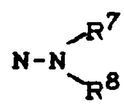

steht, worin
n für die Zahlen 0 oder 1 steht und
$R^6$ für Wasserstoff, Halogen-$C_1$-$C_8$-Alkyl für $C_1$-$C_8$-Alkyl, welches gegebenenfalls durch $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, $C_1$-$C_4$-Alkoxy-carbonyl, $C_1$-$C_4$-Alkylamino-carbonyl, Di-($C_1$-$C_4$-alkyl)-amino-carbonyl, N-($C_1$-$C_4$-alkyl)-phenylamino-carbonyl oder Cyano substituiert ist, $C_2$-$C_6$-Alkenyl, Halogen-$C_2$-$C_6$-alkenyl, $C_3$-$C_6$-Alkinyl, $C_3$-$C_6$-Cycloalkyl, $C_3$-$C_6$-Cycloalkyl-$C_1$-$C_2$-alkyl, Benzyl, Phenylethyl, Benzhydryl oder Phenyl, welche jeweils gegebenenfalls in der aromatischen Komponente durch Fluor, Chlor, Brom, Cyano, Nitro, $C_1$-$C_4$-Alkyl, $C_1$-$C_4$-Alkoxy und/oder $C_1$-$C_4$-Alkoxycarbonyl substituiert sind, steht,
$R^7$ für Wasserstoff oder $C_1$-$C_4$-Alkyl steht und
$R^8$ für Wasserstoff, Halogen-$C_1$-$C_4$-alkyl, für $C_1$-$C_4$-Alkyl, welches gegebenenfalls durch Cyano, Nitro, $C_1$-$C_4$-Alkoxy oder $C_1$-$C_4$-Alkoxy-carbonyl substituiert ist, $R^8$ weiterhin für $C_3$-$C_6$-Alkenyl, $C_3$-$C_6$-Alkinyl, $C_3$-$C_6$-Cycloalkyl, für Benzyl, Phenylethyl, Benzylhydryl oder Phenyl, welche jeweils in der aromatischen Komponente gegebenenfalls durch Fluor, Chlor, Brom, Cyano, Nitro, $C_1$-$C_4$-Alkyl, Trifluormethyl, $C_1$-$C_4$-Alkoxy, Difluormethoxy, Trifluormethoxy, $C_1$-$C_4$-Alkylthio, Trifluormethylthio oder $C_1$-$C_4$-Alkoxycarbonyl substituiert sind, $C_1$-$C_4$-Alkyl-carbonyl, $C_1$-$C_4$-Alkoxy-carbonyl, $C_1$-$C_4$-Alkylsulfonyl, Phenylsulfonyl oder Naphthylsulfonyl, wobei Phenylsulfonyl oder Naphthylsulfonyl gegebenenfalls durch Fluor, Chlor, Brom, Cyano, Nitro, $C_1$-$C_4$-Alkyl, Trifluormethyl, $C_1$-$C_4$-Alkoxy, Difluormethoxy, Trifluormethoxy, $C_1$-$C_4$-Alkylthio, Trifluormethylthio und/oder $C_1$-$C_4$-Alkoxy-carbonyl substituiert sind, oder $R^8$ für Pyrimidinyl steht.

3. Disubstituierte Naphthaline der Formel (I) gemäß Anspruch 1,
in welcher
A für Methylen ($-CH_2-$), Dimethylen ($-CH_2CH_2-$), Trimethylen ($-CH_2CH_2CH_2-$), Ethyliden

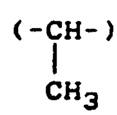

oder Propyliden

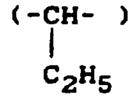

steht,

R für Methyl, Ethyl, Propyl, Isopropyl, Butyl, Isobutyl, sec-Butyl oder tert-Butyl steht,
$R^1$ für Cyano, Fluor oder Chlor steht,
$R^2$ für Wasserstoff, Fluor oder Chlor steht,
$R^3$ für Chlor, Trifluormethyl oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor oder Chlor steht,
Y für Sauerstoff oder Schwefel steht, und
Z für Sauerstoff, die Gruppierung N-$(O)_n$-$R^6$ oder

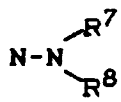

steht, worin

n für die Zahlen 0 oder 1 steht und
$R^6$ für Wasserstoff, $C_1$-$C_4$-Alkyl, $C_3$-$C_4$-Alkenyl, $C_1$-$C_3$-Alkoxy-carbonyl-$C_1$-$C_2$-alkyl, $C_1$-$C_3$-Alkylamino-carbonyl-$C_1$-$C_2$-alkyl, Di-($C_1$-$C_3$-alkyl)-amino-carbonyl-$C_1$-$C_2$-alkyl, N-($C_1$-$C_3$-Alkyl)-phenylamino-carbonyl-$C_1$-$C_2$-alkyl oder Benzyl, welches gegebenenfalls in der aromatischen Komponente durch Fluor, Chlor, Methyl, Methoxy und/oder Methoxycarbonyl substituiert ist, steht,
$R^7$ für Wasserstoff oder Methyl steht und
$R^8$ für $C_1$-$C_4$-Alkyl, Phenyl, welches gegebenenfalls durch Fluor, Chlor, Brom, Methyl, Ethyl, Trifluormethyl, Methoxy, Ethoxy, Difluormethoxy und/oder Trifluormethoxy substituiert ist, Acetyl, Propionyl, Methoxycarbonyl, Ethoxycarbonyl, Methylsulfonyl, Ethylsulfonyl, Phenylsulfonyl oder Tolylsulfonyl steht.

**4.** Verfahren zur Herstellung von disubstituierten Naphthalinen der Formel (I)

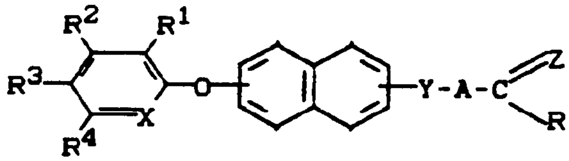
(I)

in welcher
A für gegebenenfalls verzweigtes Alkandiyl steht,
R für Wasserstoff oder für jeweils gegebenenfalls substituiertes Alkyl oder Phenyl steht,
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht,
X für Stickstoff oder die Gruppierung C-$R^5$ steht, worin
$R^5$ für Wasserstoff oder Halogen steht,
Y für Sauerstoff oder Schwefel steht, und
Z für Sauerstoff, die Gruppierung N-$(O)_n$-$R^6$ oder

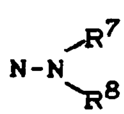

steht, worin

n für die Zahlen 0 oder 1 steht und

$R^6$ für Wasserstoff oder für einen gegebenenfalls substituierten Rest aus der Reihe Alkyl, Alkenyl, Alkinyl, Cycloalkyl, Cycloalkylalkyl, Aralkyl und Aryl steht,

$R^7$ für Wasserstoff oder Alkyl steht und

$R^8$ für Wasserstoff oder einen gegebenenfalls substituierten Rest aus der Reihe Alkyl, Alkenyl, Alkinyl, Cyclalkyl, Aralkyl, Aryl, Alkylcarbonyl, Alkoxycarbonyl, Alkylsulfonyl, Arylsulfonyl und Heteroaryl steht,

dadurch gekennzeichnet, daß man

(a) substituierte Hydroxy- bzw. Mercaptonaphthaline der allgemeinen Formel (II)

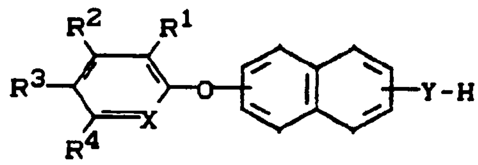

(II)

in welcher

$R^1$, $R^2$, $R^3$, $R^4$, X und Y die oben angegebenen Bedeutungen haben,

mit Alkylierungsmitteln der allgemeinen Formel (III)

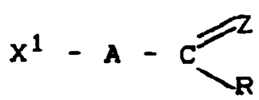

(III)

in welcher

A, R und Z die oben angegebenen Bedeutungen haben und

$X^1$ für Halogen steht,

- im Fall, daß Z für Sauerstoff steht, gegebenenfalls mit entsprechenden Dimethyl- oder Diethyl-acetalen bzw. -ketalen -

gegebenenfalls in Gegenwart eines Säureakzeptors und gegebenenfalls in Gegenwart eines Verdünnungsmittels umsetzt, oder daß man

(b) für den Fall, daß Z für die Gruppierung $N\text{-}(O)_n\text{-}R^6$ oder für die Gruppierung

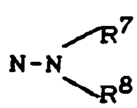

steht,

entsprechende Carbonylverbindungen der Formel (Ia)

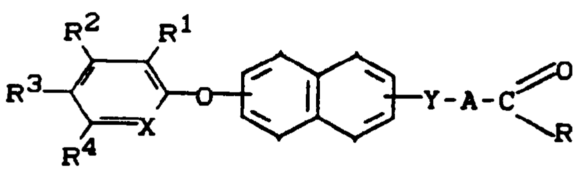

(Ia)

in welcher

A, R, $R^1$, $R^2$, $R^3$, $R^4$, X und Y die oben angegebenen Bedeutungen haben,

mit Aminoverbindungen der Formeln (IV) oder (V)

$H_2N-(O)_n-R^6$

(IV)

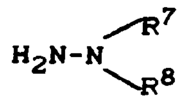

(V)

worin

n, $R^6$, $R^7$ und $R^8$ die oben angegebenen Bedeutungen haben,
oder mit deren Hydrochloriden

gegebenenfalls in Gegenwart eines Katalysators und gegebenenfalls in Gegenwart eines Verdünnungsmittels umsetzt, oder daß man

(c) für den Fall, daß Z für die Gruppierung N-O-$R^6$ steht,
entsprechende Hydroximinoverbindungen der Formel (Ib)

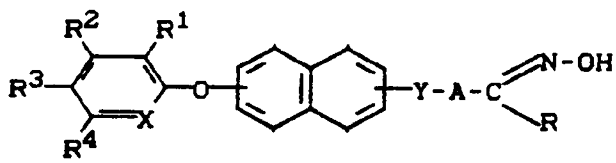

(Ib)

in welcher
A, R, $R^1$, $R^2$, $R^3$, $R^4$, X und Y die oben angegebenen Bedeutungen haben,
mit Alkylierungsmitteln der Formel (VI)

$$X^2 - R^6 \quad (VI)$$

in welcher

$R^6$ die oben angegebene Bedeutung hat und
$X^2$ für Halogen, Alkylsulfonyloxy oder Arylsulfonyloxy steht,
gegebenenfalls in Gegenwart eines Verdünnungsmittels umsetzt.

5. Herbizide Mittel, gekennzeichnet durch einen Gehalt an mindestens einem disubstituierten Naphthalin der Formel (I) gemäß den Ansprüchen 1 bis 4.

6. Verfahren zur Bekämpfung von Unkräutern, dadurch gekennzeichnet, daß man disubstituierte Naphthaline der Formel (I) gemäß den Ansprüchen 1 bis 4 auf die Unkräuter und/oder ihren Lebensraum einwirken läßt.

7. Verwendung von disubstituierten Naphthalinen der Formel (I) gemäß den Ansprüchen 1 bis 4 zur Bekämpfung von Unkräutern.

8. Verfahren zur Herstellung von herbiziden Mitteln, dadurch gekennzeichnet, daß man disubstituierte Naphthaline der Formel (I) gemäß den Ansprüchen 1 bis 4 mit Streckmitteln und/oder oberflächenaktiven Substanzen vermischt.

## Claims

1. Disubstituted naphthalenes of the formula (I)

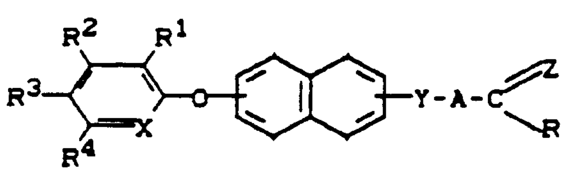

(I)

in which
A represents optionally branched alkanediyl,
R represents hydrogen or in each case optionally substituted alkyl or phenyl,
$R^1$ represents hydrogen, halogen, cyano or trifluoromethyl,
$R^2$ represents hydrogen or halogen,
$R^3$ represents halogen, trifluoromethyl, trifluoromethoxy, trifluoromethylthio or trifluoromethylsulphonyl,
$R^4$ represents hydrogen or halogen,
X represents nitrogen or the group C-$R^5$, where
$R^5$ represents hydrogen or halogen,
Y represents oxygen or sulphur, and
Z represents oxygen, the group N-$(O)_n$-$R^6$ or

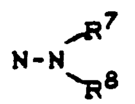 ,

where
n represents the numbers 0 or 1 and
$R^6$ represents hydrogen or an optionally substituted radical from the series comprising alkyl, alkenyl, alkinyl, cycloalkyl, cycloalkylalkyl, aralkyl and aryl,
$R^7$ represents hydrogen or alkyl and
$R^8$ represents hydrogen or an optionally substituted radical from the series comprising alkyl, alkenyl, alkinyl, cyclalkyl, aralkyl, aryl, alkylcarbonyl, alkoxycarbonyl, alkylsulphonyl, arylsulphonyl and heteroaryl.

**2.** Disubstituted naphthalenes of the formula (I) according to Claim 1,
in which
A represents optionally branched $C_1$-$C_4$-alkanediyl,
R represents hydrogen, $C_1$-$C_6$-alkyl or phenyl,
$R^1$ represents hydrogen, fluorine, chlorine, bromine, cyano or trifluoromethyl,
$R^2$ represents hydrogen, fluorine or chlorine,
$R^3$ represents fluorine, chlorine, bromine, trifluoromethyl, trifluoromethoxy, trifluoromethylthio or trifluoromethylsulphonyl,
$R^4$ represents hydrogen, fluorine or chlorine,
X represents nitrogen or the group C-$R^5$ where
$R^5$ represents hydrogen, fluorine, chlorine or bromine,
Y represents oxygen or sulphur, and
Z represents oxygen, the group N-$(O)_n$-$R^6$ or

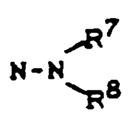 ,

where
n represents the numbers 0 or 1 and
$R^6$ represents hydrogen, halogeno-$C_1$-$C_8$-akyl, or represents $C_1$-$C_8$-alkyl which is optionally substituted by $C_1$-$C_4$-alkoxy, $C_1$-$C_4$-alkylthio, $C_1$-$C_4$-alkylsulphinyl, $C_1$-$C_4$-alkylsulphonyl, carboxyl, $C_1$-$C_4$-alkoxycarbonyl, $C_1$-$C_4$-alkylamino-carbonyl, di-($C_1$-$C_4$-alkyl)-amino-carbonyl, N-($C_1$-$C_4$-alkyl)-phenylamino-carbonyl or cyano, $C_2$-$C_6$-alkenyl, halogeno-$C_2$-$C_6$-alkenyl, $C_3$-$C_6$-alkinyl, $C_3$-$C_6$-cycloalkyl, $C_3$-$C_6$-cycloalkyl-$C_1$-$C_2$-alkyl, or benzyl, phenylethyl, benzhydryl or phenyl which are in each case optionally substituted in the aromatic moiety by fluorine, chlorine, bromine, cyano, nitro, $C_1$-$C_4$-alkyl, $C_1$-$C_4$-alkoxy and/or $C_1$-$C_4$-alkoxycarbonyl,
$R^7$ represents hydrogen or $C_1$-$C_4$-alkyl and
$R^8$ represents hydrogen, halogeno-$C_1$-$C_4$-alkyl, or represents $C_1$-$C_4$-alkyl which is optionally substituted by cyano, nitro, $C_1$-$C_4$-alkoxy or $C_1$-$C_4$-alkoxy-carbonyl, $R^8$ furthermore represents $C_3$-$C_6$-al-

kenyl, $C_3$-$C_6$-alkinyl, $C_3$-$C_6$-cycloalkyl, or represents benzyl, phenylethyl, benzylhydryl or phenyl, which are in each case optionally substituted in the aromatic moiety by fluorine, chlorine, bromine, cyano, nitro, $C_1$-$C_4$-alkyl, trifluoromethyl, $C_1$-$C_4$-alkoxy, difluoromethoxy, trifluoromethoxy, $C_1$-$C_4$-alkylthio, trifluoromethylthio or $C_1$-$C_4$-alkoxy-carbonyl, or is $C_1$-$C_4$-alkyl-carbonyl, $C_1$-$C_4$-alkoxy-carbonyl, $C_1$-$C_4$-alkylsulphonyl, or phenylsulphonyl or naphthylsulphonyl, where phenylsulphonyl or naphthylsulphonyl are optionally substituted by fluorine, chlorine, bromine, cyano, nitro, $C_1$-$C_4$-alkyl, trifluoromethyl, $C_1$-$C_4$-alkoxy, difluoromethoxy, trifluoromethoxy, $C_1$-$C_4$-alkylthio, trifluoromethylthio and/or $C_1$-$C_4$-alkoxy-carbonyl, or $R^8$ represents pyrimidinyl.

3. Disubstituted naphthalenes of the formula (I) according to Claim 1,
in which
A represents methylene ($-CH_2-$), dimethylene ($-CH_2CH_2-$), trimethylene ($-CH_2CH_2CH_2-$), ethylidene

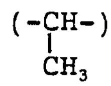

or propylidene

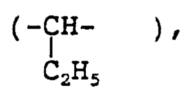

R represents methyl, ethyl, propyl, isopropyl, butyl, isobutyl, sec-butyl or tert-butyl,
$R^1$ represents cyano, fluorine or chlorine,
$R^2$ represents hydrogen, fluorine or chlorine,
$R^3$ represents chlorine, trifluoromethyl or trifluoromethylsulphonyl,
$R^4$ represents hydrogen, fluorine or chlorine,
X represents nitrogen or the group C-$R^5$, where
$R^5$ represents hydrogen, fluorine or chlorine,
Y represents oxygen or sulphur, and
Z represents oxygen, the group N-$(O)_n$-$R^6$ or

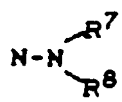 ,

where
n represents the numbers 0 or 1 and
$R^6$ represents hydrogen, $C_1$-$C_4$-alkyl, $C_3$-$C_4$-alkenyl, $C_1$-$C_3$-alkoxy-carbonyl-$C_1$-$C_2$-alkyl, $C_1$-$C_3$-alkylamino-carbonyl-$C_1$-$C_2$-alkyl, di-($C_1$-$C_3$-alkyl)-amino-carbonyl-$C_1$-$C_2$-alkyl, N-($C_1$-$C_3$-alkyl)-phenyl amino-carbonyl -$C_1$-$C_2$-alkyl, or benzyl which is optionally substituted in the aromatic moiety by fluorine, chlorine, methyl, methoxy and/or methoxycarbonyl,
$R^7$ represents hydrogen or methyl and
$R^8$ represents $C_1$-$C_4$-alkyl, phenyl which is optionally substituted by fluorine, chlorine, bromine, methyl, ethyl, trifluoromethyl, methoxy, ethoxy, difluoromethoxy and/or trifluoromethoxy, or represents acetyl, propionyl, methoxycarbonyl, ethoxycarbonyl, methylsulphonyl, ethylsulphonyl, phenylsulphonyl or tolylsulphonyl.

4. Process for the preparation of disubstituted naphthalenes of the formula (I)

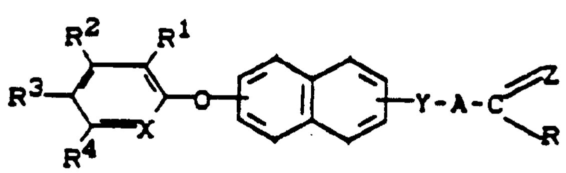 (I)

in which

A represents optionally branched alkanediyl,
R represents hydrogen or in each case optionally substituted alkyl or phenyl,
$R^1$ represents hydrogen, halogen, cyano or trifluoromethyl,
$R^2$ represents hydrogen or halogen,
$R^3$ represents halogen, trifluoromethyl, trifluoromethoxy, trifluoromethylthio or trifluoromethylsulphonyl,
$R^4$ represents hydrogen or halogen,
X represents nitrogen or the group C-$R^5$, where
$R^5$ represents hydrogen or halogen,
Y represents oxygen or sulphur, and
Z represents oxygen, the group N-$(O)_n$-$R^6$ or

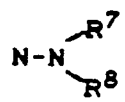 ,

where

n represents the numbers 0 or 1 and
$R^6$ represents hydrogen or an optionally substituted radical from the series comprising alkyl, alkenyl, alkinyl, cycloalkyl, cycloalkylalkyl, aralkyl and aryl,
$R^7$ represents hydrogen or alkyl and
$R^8$ represents hydrogen or an optionally substituted radical from the series comprising alkyl, alkenyl, alkinyl, cycloalkyl, aralkyl, aryl, alkylcarbonyl, alkoxycarbonyl, alkylsuphonyl, arylsulphonyl and heteroaryl,

characterized in that

(a) substituted hydroxy- or mercaptonaphthalenes of the general formula (II)

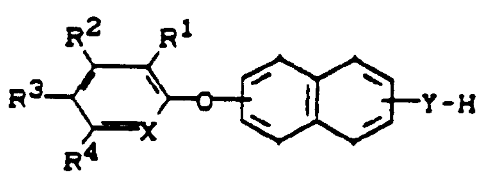 (II)

in which
$R^1$, $R^2$, $R^3$, $R^4$, X and Y have the abovementioned meanings
are reacted with alkylating agents of the general formula (III)

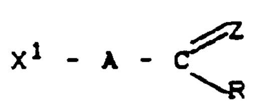 (III)

in which
A, R and Z have the abovementioned meanings and
$X^1$ represents halogen,

- in the event that Z represents oxygen, if appropriate with corresponding dimethyl or diethyl acetals or ketals -

if appropriate in the presence of an acid acceptor and if appropriate in the presence of a diluent, or in that

(b) in the event that Z represents the group N-$(O)_n$-$R^6$ or the group

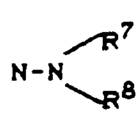

corresponding carbonyl compounds of the formula (Ia)

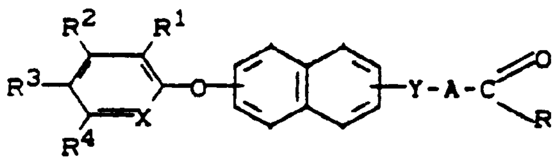

(Ia)

in which
A, R, $R^1$, $R^2$, $R^3$, $R^4$, X and Y have the abovementioned meanings
are reacted with amino compounds of the formulae ( IV) or (V)

$H_2N-(O)_n-R^6$

(IV)

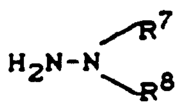

(V)

where
n, $R^6$, $R^7$ and $R^8$ have the abovementioned meanings,
or with the hydrochlorides thereof,
if appropriate in the presence of a catalyst and if appropriate in the presence of a diluent, or in that
(c) in the event that Z represents the group N-O-$R^6$,
corresponding hydroximino compounds of the formula (Ib)

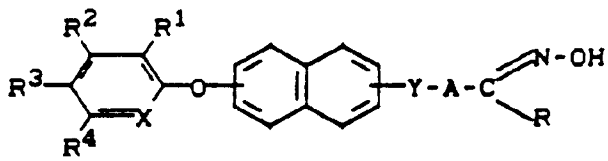

(Ib)

in which
A, R, $R^1$, $R^2$, $R^3$, $R^4$, X and Y have the abovementioned meanings
are reacted with alkylating agents of the formula (VI)

$$X^2 - R^6 \quad (VI)$$

in which
$R^6$ has the abovementioned meaning and
$X^2$ represents halogen, alkylsulphonyloxy or arylsulphonyloxy,
if appropriate in the presence of a diluent.

5. Herbicidal agent, characterized in that it contains at least one disubstituted naphthalene of the formula (I) according to Claims 1 to 4.

6. Method of combating weeds, characterized in that disubstituted naphthalenes of the formula (I) according to Claims 1 to 4 are allowed to act on the weeds and/or their environment.

7. Use of disubstituted naphthalenes of the formula (I) according to Claims 1 to 4 for combating weeds.

8. Process for the preparation of herbicidal agents, characterized in that disubstituted naphthalenes of the formula (I) according to Claims 1 to 4 are mixed with extenders and/or surface-active substances.

## Revendications

1. Naphtalènes disubstitués de formule (I)

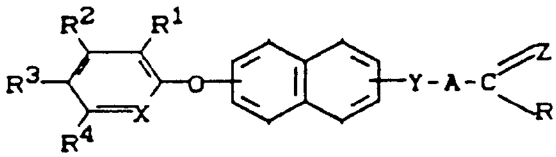

(I)

dans laquelle

A représente un groupe alcanediyle éventuellement ramifié,

R est l'hydrogène ou un groupe alkyle ou groupe phényle, chacun éventuellement substitué

$R^1$ est l'hydrogène, un halogène, un groupe cyano ou trifluorométhyle,

$R^2$ est l'hydrogène ou un halogène,

$R^3$ est un halogène, un groupe trifluorométhyle, trifluorométhoxy, trifluorométhylthio ou trifluorométhylsulfonyle,

$R^4$ est l'hydrogène ou un halogène,

X est l'azote ou le groupement C-$R^5$, dans lequel $R^5$ est l'hydrogène ou un halogène,

Y est l'oxygène ou le soufre et

Z est l'oxygène, le groupement N-$(O)_n$-$R^6$ ou

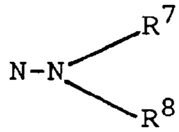

,

dans lequel

n représente les nombres 0 ou 1 et

$R^6$ est l'hydrogène ou un reste, éventuellement substitué, de la série alkyle, alcényle, alcynyle, cycloalkyle, cycloalkylalkyle, aralkyle et aryle,

$R^7$ est l'hydrogène ou un groupe alkyle et

$R^8$ est l'hydrogène ou un reste, éventuellement substitué, de la série alkyle, alcényle, alcynyle, cycloalkyle, aralkyle, aryle, alkylcarbonyle, alkoxycarbonyle, alkylsulfonyle, arylsulfonyle et hétéroaryle.

**2.** Naphtalènes disubstitués de formule (I) suivant la revendication 1, dans lesquels :

A représente un groupe alcanediyle en $C_1$ à $C_4$ éventuellement substitué,

R est l'hydrogène, un groupe alkyle en $C_1$ à $C_6$ ou phényle,

$R^1$ est l'hydrogène, le fluor, le chlore, le brome, un groupe cyano ou trifluorométhyle,

$R^2$ est l'hydrogène, le fluor ou le chlore,

$R^3$ est le fluor, le chlore, le brome, un groupe trifluorométhyle, trifluorométhoxy, trifluorométhylthio ou trifluorométhylsulfonyle,

$R^4$ est l'hydrogène, le fluor ou le chlore,

X représente l'azote ou le groupement C-$R^5$, dans lequel

$R^5$ est l'hydrogène, le fluor, le chlore ou le brome,

Y est l'oxygène ou le soufre, et

Z est l'oxygène, le groupement N-$(O)_n$-$R^6$ ou

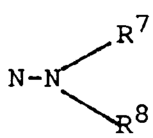

,

dans lequel

n représente les nombres 0 ou 1 et

$R^6$ est l'hydrogène, un groupe halogénalkyle en $C_1$ à $C_8$, un groupe alkyle en $C_1$ à $C_8$ qui porte facultativement un substituant alkoxy en $C_1$ à $C_4$, alkylthio en $C_1$ à $C_4$, alkyl sulfinyle en $C_1$ à $C_4$, alkylsulfonyle en $C_1$ à $C_4$, carboxy, (alkoxy en $C_1$ à $C_4$) carbonyle, (alkylamino en $C_1$ à $C_4$) carbonyle, di(alkyle en $C_1$ à $C_4$)aminocarbonyle, N-(alkyle en $C_1$ à $C_4$) phénylaminocarbonyle ou cyano, un groupe

alcényle en $C_2$ à $C_6$, halogénalcényle en $C_2$ à $C_6$, alcynyle en $C_3$ à $C_6$, cycloalkyle en $C_3$ à $C_6$, (cycloalkyle en $C_3$ à $C_6$)-(alkyle en $C_1$ ou $C_2$), un groupe benzyle, phényléthyle, benzhydryle ou phényle, ces groupes étant substitués chacun le cas échéant dans le composant aromatique par du fluor, du chlore, du brome, un radical cyano, nitro, alkyle en $C_1$ à $C_4$, alkoxy en $C_1$ à $C_4$ et/ou (alkoxy en $C_1$ à $C_4$)carbonyle,

$R^7$ représente l'hydrogène ou un groupe alkyle en $C_1$ à $C_4$, et

$R^8$ représente l'hydrogène, un groupe halogénalkyle en $C_1$ à $C_4$, un groupe alkyle en $C_1$ à $C_4$ qui porte éventuellement un substituant cyano, nitro, alkoxy en $C_1$ à $C_4$ ou (alkoxy en $C_1$ à $C_4$)carbonyle,

$R^8$ représente en outre un groupe alcényle en $C_3$ à $C_6$, alcynyle en $C_3$ à $C_6$, cycloalkyle en $C_3$ à $C_6$, un groupe benzyle, phényléthyle, benzyhydryle ou phényle, chacun pouvant être substitué dans le composant aromatique éventuellement par du fluor, du chlore, du brome, un radical cyano, nitro, alkyle en $C_1$ à $C_4$, trifluorométhyle, alkoxy en $C_1$ à $C_4$, difluorométhoxy, trifluorométhoxy, alkylthio en $C_1$ à $C_6$, trifluorométhylthio ou (alkoxy en $C_1$ à $C_4$)-carbonyle, un groupe (alkyle en $C_1$ à $C_4$)-carbonyle, (alkoxy en $C_1$ à $C_4$)-carbonyle, alkylsulfonyle en $C_1$ à $C_4$, phénylsulfonyle ou naphtylsulfonyle, le groupe phénylsulfonyle ou naphtylsulfonyle étant substitué le cas échéant par du fluor, du chlore, du brome, un radical cyano, nitro, alkyle en $C_1$ à $C_4$, trifluorométhyle, alkoxy en $C_1$ à $C_4$, di-fluorométhoxy, trifluorméthoxy, alkylthio en $C_1$ à $C_4$, trifluorométhylthio et/ou (alkoxy en $C_1$ à $C_4$)-carbonyle, ou bien $R^8$ est un groupe pyrimidinyle.

**3.** Naphtalènes disubstitués de formule (I) suivant la revendication 1, dans laquelle

A est un groupe méthylène ($-CH_2$), diméthylène ($-CH_2CH_2-$), triméthylène ($-CH_2CH_2CH_2-$), éthylidène

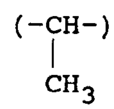

ou propylidène

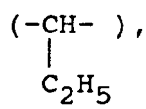

R est un groupe méthyle, éthyle, propyle, isopropyle, butyle, isobutyle, sec.-butyle ou tertiobutyle,
$R^1$ représente un groupe cyano, du fluor ou du chlore,
$R^2$ est l'hydrogène, le fluor ou le chlore,
$R^3$ représente du chlore ou un groupe trifluorométhyle ou trifluorométhylsulfonyle,
$R^4$ est l'hydrogène, le fluor ou le chlore,
X est de l'azote ou le groupement C-$R^5$, dans lequel
$R^5$ représente l'hydrogène, le fluor ou le chlore,
Y est l'oxygène ou le soufre, et
Z est l'oxygène, le groupement N-$(O)_n$-$R^6$ ou

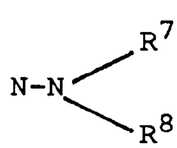

dans lequel

n représente les nombres 0 ou 1 et

$R^6$ est l'hydrogène, un groupe alkyle en $C_1$ à $C_4$, alcényle en $C_3$ ou $C_4$, (alkoxy en $C_1$ à $C_3$)-carbonyl-(alkyle en $C_1$ ou $C_2$), (alkylamino en $C_1$ à $C_3$)-carbonyl-(alkyle en $C_1$ ou $C_2$), di(alkyle en $C_1$ à $C_3$)amino-carbonyl-(alkyle en $C_1$ ou $C_2$), N-(alkyle en $C_1$ à $C_3$)phénylaminocarbonyl-(alkyle en $C_1$ ou $C_2$) ou benzyle, qui est substitué le cas échéant dans le composant aromatique par du fluor, du chlore, un radical méthyle, méthoxy et/ou méthoxycarbonyle,

$R^7$ est l'hydrogène ou le groupe méthyle et

$R^8$ est un groupe alkyle en $C_1$ à $C_4$, phényle qui est substitué le cas échéant par du fluor, du chlore, du brome, un radical méthyle, éthyle, trifluorométhyle, méthoxy, éthoxy, difluorométhoxy et/ou trifluorométhoxy, un groupe acétyle, propionyle, méthoxycarbonyle, éthoxycarbonyle, méthylsulfonyle, éthylsulfonyle, phénylsulfonyle ou tolylsulfonyle.

4. Procédé de production de naphtalènes disubstitués de formule (I)

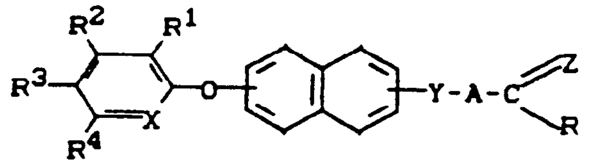

(I)

dans laquelle

A est un groupe alcanediyle éventuellement substitué,

R est l'hydrogène ou un groupe alkyle ou phényle chacun éventuellement substitué,

$R^1$ est l'hydrogène, un halogène, un groupe cyano ou trifluorométhyle,

$R^2$ est l'hydrogène ou un halogène,

$R^3$ est un halogène, un groupe trifluorométhyle, trifluorométhoxy, trifluorométhylthio ou trifluorométhylsulfonyle,

$R^4$ est l'hydrogène ou un halogène,

X est l'azote ou le groupement C-$R^5$, où $R^5$ représente l'hydrogène ou un halogène,

Y est l'hydrogène ou le soufre, et

Z est l'oxygène, le groupement N-(O)-$_n$-$R^6$ ou

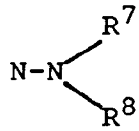

dans lequel

n représente les nombres 0 ou 1 et

$R^6$ est l'hydrogène ou un reste éventuellement substitué de la série alkyle, alcényle, alcynyle, cycloalkyle, cycloalkylalkyle, aralkyle et aryle,

$R^7$ est l'hydrogène ou un groupe alkyle et

$R^8$ est l'hydrogène ou un reste éventuellement substitué de la série alkyle, alcényle, alcynyle, cycloalkyle, aralkyle, aryle, alkylcarbonyle, alkoxycarbonyle, alkylsulfonyle, arylsulfonyloe et hétéroaryle, caractérisé en ce que

(a) on fait réagir des hydroxy- ou mercaptonaphtalènes substitués de formule générale (II)

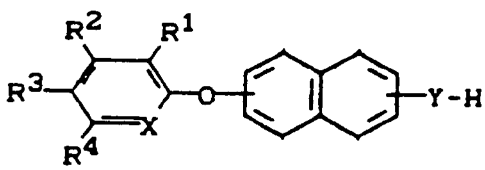

(II)

dans laquelle

$R^1$, $R^2$, $R^3$, $R^4$, X et Y ont les définitions indiquées ci-dessus,

avec des agents alkylants de formule générale (III)

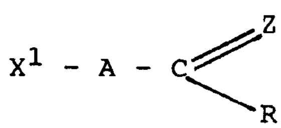

(III)

dans laquelle

A, R et Z ont les définitions indiquées ci-dessus et
$X^1$ représente un halogène,
- au cas où Z représente l'oxygène, éventuellement avec des diméthyl- ou des diéthylacétals ou -cétals correspondants, le cas échéant en présence d'un accepteur d'acide et en la présence éventuelle d'un diluant, ou bien
(b) au cas où Z représente le groupement N-$(O)_n$-$R^6$ ou le groupement

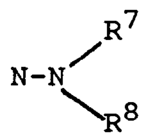,

on fait réagir des composés carbonyliques correspondants de formule (Ia)

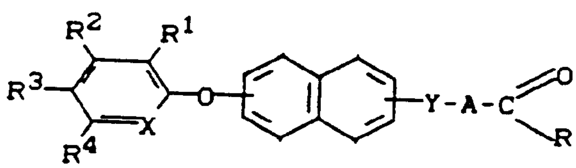 (Ia)

dans laquelle
A, R, $R^1$, $R^2$, $R^3$, $R^4$, X et Y ont les définitions indiquées ci-dessus,
avec des composés aminés de formules (IV) ou (V)

$H_2N-(O)_n-R^6$

(IV)

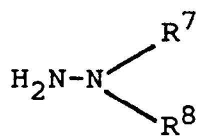

(V)

où
n, $R^6$, $R^7$ et $R^8$ ont les définitions indiquées ci-dessus
ou avec leurs chlorhydrates
le cas échéant en présence d'un catalyseur et en la présence éventuelle d'un diluant, ou bien
(c) au cas où Z représente le groupement N-O-$R^6$, on fait réagir des composés hydroxy-imino correspondants de formule (Ib)

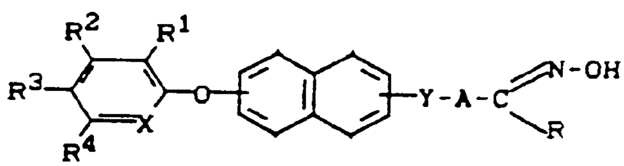 (Ib)

dans laquelle
A, R, $R^1$, $R^2$, $R^3$, $R^4$, X et Y ont les définitions indiquées ci-dessus,
avec des agents alkylants de formule (VI)

$$X^2 - R^6 \quad (VI)$$

dans laquelle
$R^6$ a la définition indiquée ci-dessus et
$X^2$ est un halogène, un groupe alkylsulfonyloxy ou arylsulfonyloxy,
le cas échéant en présence d'un diluant.

5. Compositions herbicides, caractérisées par une teneur en au moins un naphtalène disubstitué de formule

(I) suivant les revendications 1 à 4.

6. Procédé pour combattre des mauvaises herbes, caractérisé en ce qu'on fait agir les naphtalènes disubstitués de formule (I) suivant les revendications 1 à 4 sur les mauvaises herbes et/ou sur leur milieu.

7. Utilisation de naphtalènes disubstitués de formule (I) suivant les revendications 1 à 4 pour combattre des mauvaises herbes.

8. Procédé de préparation de compositions herbicides, caractérisé en ce qu'on mélange des naphtalènes disubstitués de formule (I) suivant les revendications 1 à 4 avec des diluants et/ou des substances tensioactives.